(19) Europäisches Patentamt
European Patent Office
Office européen des brevets

(11) **EP 1 273 922 B1**

(12) **EUROPEAN PATENT SPECIFICATION**

(45) Date of publication and mention
of the grant of the patent:
**23.05.2007 Bulletin 2007/21**

(51) Int Cl.:
***G01R 19/00*** *(2006.01)*  ***A61B 5/055*** *(2006.01)*
***A61B 5/0476*** *(2006.01)*

(21) Application number: **01116436.5**

(22) Date of filing: **06.07.2001**

(54) **Methods and devices for measuring electrical currents**

Gerät und Verfahren zur Strommessung

Appareil et procédé de mesure de courant

(84) Designated Contracting States:
**DE FR GB NL**

(43) Date of publication of application:
**08.01.2003 Bulletin 2003/02**

(73) Proprietor: **Max-Planck-Gesellschaft zur Förderung**
**der Wissenschaften e.V.**
**80539 München (DE)**

(72) Inventor: **Oeltermann, Axel**
**72076 Tübingen (DE)**

(74) Representative: **Hertz, Oliver**
**v. Bezold & Partner**
**Patentanwälte**
**Akademiestrasse 7**
**80799 München (DE)**

(56) References cited:
**EP-A- 0 477 501**  **WO-A-00/27279**
**DE-A- 4 101 556**  **DE-A- 4 447 538**
**DE-A- 19 626 292**  **US-A- 4 640 290**
**US-A- 4 689 305**  **US-A- 5 245 286**
**US-A- 5 371 472**  **US-A- 5 445 162**

- **P. HOROWITZ AND W HILL: "The Art of Electronics" 1989 , CAMBRIDGE UNIVERSITY PRESS , CAMBRIDGE, NEW YORK XP002187675 * page 468; figure I ***
- **IWANSSON ET AL.: "Handbook of Sensors and Actuators 7: Measuring Current, Voltage and power" 1999 , ELSEVIER , AMSTERDAM XP002187550 * page 110, paragraph 1 ***
- **HESS A ET AL: "Correlation- versus integration-analysis implications for functional magnetic resonance imaging and optical recording of intrinsic signals (ORIS)" IMAGE PROCESSING, 1999. ICIP 99. PROCEEDINGS. 1999 INTERNATIONAL CONFERENCE ON KOBE, JAPAN 24-28 OCT. 1999, PISCATAWAY, NJ, USA, IEEE, US, 24 October 1999 (1999-10-24), pages 426-429, XP010368834 ISBN: 0-7803-5467-2**

## Description

The present invention concerns methods and devices for measuring electrical currents in the presence of strong fields, as in magnetic field gradients.

The problem of measuring small dc or ac currents via long connection lines or injecting small currents into long lines is generally known. Due to the capacitance of the line, a portion of the current is absorbed, so that the measuring result or the current injection is effectively deteriorated. In the present context, small currents are currents, which are changed due to the line capacitance depending in particular on the length of the line.

Small currents are generated or used e.g. with detectors or sensors (as e.g. photodiodes, currents sensors etc.), in an experimental setup or in a technological process. Additional problems arise, if the currents have to be measured or injected in the presence of strong disturbing fields or influences, e. g in NMR, cooling or vacuum devices.

An example for measurements in the presence of strong disturbing fields is given with Magnetic Resonance procedures causing a series of problems that are illustrated in the following.

Since its inception so-called functional <u>Magnetic Resonance Imaging</u> (BOLD fMRI) has generated a great deal of excitement not only as a brain activation mapping tool, but also as a means of studying the dynamics of neural networks by tracking fMRI response characteristics across various spatial and temporal scales (see Ogawa,S. & Lee,T.M. Magnetic resonance imaging of blood vessels at high fields: in vivo and in vitro measurements and image simulation. Magnetic Resonance in Medicine 16, 9-18 (1990); Bandettini,P.A., Wong,E.C., Hinks,R.S., Tikofsky,R.S. & Hyde,J.S. Time course EPI of human brain function during task activation. Magnetic Resonance in Medicine 25, 390-397 (1992); Frahm, J., Bruhn,H., Merboldt,K.D. & Hanicke, W. Dynamic MR imaging of human brain oxygenation during rest and photic stimulation. Journal of Magnetic Resonance Imaging 2, 501-505 (1992); Menon,R.S. et al. Functional brain mapping using magnetic resonance imaging. Signal changes accompanying visual stimulation. Investigative Radiology 27, Suppl-53 (1992); Kwong,K.K. et al. Dynamic magnetic resonance imaging of human brain activity during primary sensory stimulation. Proceedings of the National Academy of Sciences of the United States of America 89, 5675-5679 (1992)). Yet BOLD can only measure hemodynamic changes, such as alterations in flow, blood volume, or intravascular magnetic susceptibility, leaving many unanswered questions concerning the relationship between such cerebral hemodynamic changes and actual neural activation.

A number of studies in humans or animals have combined fMRI with electroencephalography (EEG) or optical imaging recordings of intrinsic signals (see Menon,V., Ford,J.M., Lim,K.O., Glover,G.H. & Pfefferbaum, A. Combined event-related fMRI and EEG evidence for temporal- parietal cortex activation during target detection. NeuroReport 8, 3029-3037 (1997); Krakow,K. et al. EEG recording during fMRI experiments: image quality. Human Brain Mapping 10, 10-15 (2000); Krakow,K. et al. EEG-triggered functional MRI of interictal epileptiform activity in patients with partial seizures. Brain 122, 1679-1688 (1999); Bonmassar,G., Anami,K., Ives,J. & Belliveau,J.W. Visual evoked potential (VEP) measured by simultaneous 64-channel EEG and 3T fMRI. NeuroReport 10, 1893-1897 (1999); Hess,A., Stiller,D., Kaulisch,T., Heil,P. & Scheich,H. New insights into the hemodynamic blood oxygenation level-dependent response through combination of functional magnetic resonance imaging and optical recording in gerbil barrel cortex. Journal of Neuroscience 20, 3328-3338 (2000)).

However, optical imaging studies essentially also measure hemodynamic responses (see Bonhoeffer, T. & Grinvald,A. Brain Mapping, The Methods. Toga, A.W. & Mazziotta,J.C. (eds.), pp. 55-97 (Academic Press, New York,1996)) and thus can offer very little direct evidence of the underlying neural activity, while EEG studies typically suffer from poor spatial resolution and relatively imprecise localization of the electromagnetic field patterns associated with neural current flow. It is now well established that the active states of any brain site are characterized by time-varying spatial distributions of action potentials superimposed on relatively slow varying field potentials. An ideal combination is functional imaging in experimental animals with microelectrode recordings of such potentials, including single spike responses and field potentials, whereby the latter relate well not only to spike activity but also to sub-threshold integrative processes in areas such as dendrites that are otherwise inaccessible. Microelectrode recording methods have been extensively used to obtain a wealth of data about the central nervous system in both anesthetized and alert, behaving animals. Adapting this technique for simultaneous electrophysiological and imaging experiments has therefore the very real potential to build a bridge between human studies and the large body of animal research carried out over the last 50 years; a bridge that may lead to answers unlikely to be obtained by using either technique in isolation.

The following properties of fMRI are important for experiments using anaesthetized monkeys:

> The monkey is placed in a static homogenous magnetic field with a strength of 4.7 Tesla which is created by a cylindrical, superconducting, upright coil.

The resonance frequency of the precessing protons is 200MHz in our magnet. Short periodic RF-pulses with the same centre frequency excite the protons in the brain. The emitted signal is subsequently detected with a weight on different relaxation times. The RF-coil is placed near the brain area of interest, in our case the area around the electrode tip. Therefore, the coil is placed around the chamber. How the RF-pulses cause interference problems is discussed later.

The gradient tube inside the magnet bore contains three room temperature coils, the gradient coils. Each gradient coil is able to create a magnetic field with a well defined gradient in each one of the three directions in space. These magnetic field gradients define the element of volume in the brain in which the BOLD-signal is measured. By means of certain periodic sequences of the current strength in the gradient coils it is possible to combine these elements in order to get an image of a complete brain volume, i.e. one or several slice sections through the brain with a certain thickness. In doing so, the gradients are switched with fast rising and falling ramps of the currents. We will describe later how these field gradients produce the most difficult interference problems.

The gradient coils generate interferences which are of no use for imaging. The power amplifiers which drive the gradient coils have pulse-width modulated switching output stages with a clock frequency of 81kHz. The gradient fields are modulated with this clock frequency which induces further interference problems.

Recording methods with metal-microelectrodes

The metal-microelectrodes typically used are made of sharpened platinum-iridium-wire, which is coated with glass. The tip of the wire is left uncoated and forms a small electrolytic capacitor with the tissue of the brain which has similar electrical properties as water with 0.9% NaCl (Saline). The capacity forms at the transition zone between the metal and the fluid. Therefore, the electrode acts in first approximation as a capacity and is coupled to the neuronal signal sources through this capacity. The magnitude of the capacity depends on the size of the uncoated tip. It is common practice in neurophysiology to measure the impedance of the electrode in Ohm at 1kHz to indicate the size of this capacity. Electrodes with an impedance range from e.g. 100 to $300k\Omega$ are utilized which corresponds to a capacity range from 500 to 1500pF.

The ground connection to the animal is made of a massive metal piece which is placed in the recording chamber. This chamber is made of non-conducting synthetic material and is implanted directly onto the skull. The electrode is advanced into the brain through the recording chamber and a small drilled hole in the skull. The chamber is filled with Agar which is prepared with Saline (deuterium-water+0.9% NaCl). The saline ensures the electrical connection between the ground contact and the animal. The capacity of this connection is much higher than the capacity of the electrode because here the surface of the metal-fluid boundary is much larger.

It is common practice in neurophysiology to measure the voltage between the electrode and the ground contact which has a magnitude of about $200\mu V$ peak. A voltage amplifier with a high input resistance is preferred in order not to load the high output resistance of the signal source which would diminish the signal. In the following we will show that in our case it is a better proceeding to measure current instead of voltage.

The measuring set-up is very sensitive to electric and magnetic interference fields because the output resistance of the signal source is high and the voltages to be measured are small. Therefore, booths are commonly used which are specially designed to shield the animal, the electrode and the preamplifier from undesired interference fields emitted from the surrounding area. The booths also serve as a shield against radio-frequency fields. These RF-fields are able to degrade the low-frequency recording amplifiers. Usually the cable between the electrode and the preamplifier is short. This avoids signal loss in a long cable and, at the same time, diminishes the picking-up of interference from the surrounding area.

Problems in the combination of fNMR and recording methods with metal-microelectrodes

Both measuring methods are very sensitive. The interaction of all magnetic fields must not be disturbed. This restricts strongly the choice of materials for the microelectrode, for the electrode holder and for the electrode microdrive. These materials have to be non-magnetic.

It is highly demanding to avoid the disturbance of the electrophysiological recordings emerging from the different magnetic and electric fields during imaging. Even in the normal lab environment many requirements have to be met to remove the comparatively small interferences emitted from the surroundings. The arrangements which usually have to be done to shield the recording site from inference fields were discussed above. Most of these cannot be used inside the magnet. It is not possible to shield magnetic fields nearby the electrode without a non-acceptable distortion of the magnetic field. The shielded booths mentioned above serve also to protect the electrode preamplifier from RF-radiation from the environment. In the magnet RF-pulses are emitted for imaging purpose in the immediate vicinity of the electrode and they certainly cannot be shielded.

As described above, normally, the small high-resistance signal which emerges from the electrode is fed into the preamplifier directly nearby. However, it is not possible to position a preamplifier in the magnet near the monkey head. The strong magnetic fields from the gradient coils would permeate all the electrical circuits of the preamp and make its proper function impossible. Furthermore, the RF-pulse source close to the preamp would cause problems.

The gradient fields also induce voltages in the electrode cable itself. The magnitude of the voltage depends among other things on the size of the loop area that the electrode line forms with the ground line. A common method to avoid induction voltages is to twist the lines or to use rotational symmetric cables. Doing this, the effective loop area is ideally zero and thus the induction voltage equals 0V. We use the latter method continuously. All the way until inside the recording chamber the ground line encircles the electrode and the electrode line. But it appeared that even a non-perfect rotational symmetry suffices to generates interference voltages of a not acceptable strength. In order to minimize this remainder of interference the "Near" Interference Compensation Circuit described in chapter 0 was developed.

Further problems arise from the experimental design with anaesthetized monkeys. Some additional connections to the animal are necessary in order to maintain anaesthesia, to keep the animal in the best possible physiological condition and to monitor the vital signs of the animal. These connections can transmit interference currents from outside the magnet to the animal and can as well form loops with the electrode cable. The gradient fields induce induction voltages into these loops which will superimpose to the electrode signal. It is already sufficient if these loops exist only for alternating currents via stray capacitances. Even the capacitance between the monkey and the magnet is sufficient to generate these induction voltages while the gradients are switching. Interferences of that kind are the most intensive ones. To remove these the "Far" Interference Compensation Circuit was developed.

The signal to noise ratio

The signal from the electrode is amplified, digitalised, recorded and than analysed over a large frequency band from near DC to 3kHz. We used an ADC with an input range from -10V to +10V and a resolution of 16Bit. Because the gradient interference has a structured periodic pattern it can be removed from the neuronal signal after digitalisation and recording with a mathematical approach, the principal components analysis. That can be done under the condition that the linear transmission of the gradient noise and the neuronal signal is secured all the way through from the first amplification stage to the output of the ADC. Neither the electrode amplifier nor the ADC is allowed to saturate during gradient switching.

Because without the compensation circuits the amplitude of the gradient noise is much higher than the neuronal signal it would be necessary to chose such a small amplification that the ADC does not saturate. In this case the recorded neuronal signal has such a low resolution that a lot of neuronal information is lost. Therefore, two compensation techniques were developed to minimize the gradient noise before the electrode signal gets amplified. Then the amplification can be set as high as possible but to a value that will not result in the saturation of the ADC for sure.

The performance of the compensation circuits is partly so effective that the neuronal signal and the remaining gradient noise have about the same amplitude. Even if this could not be achieved in an experiment it was always possible to remove all gradient noise with the subsequent mathematical procedure, and the yielded neuronal signal still contained all the relevant information for the later analysis.

There is a need to develop the hardware and software required for simultaneous imaging and electrophysiology recordings. Traditional methods of recording voltage changes at the electrode tip do not work within the magnet. In such recordings, a preamplifier, is typically mounted as close to the electrode as possible to minimize interference. Within the magnet, however, the switching of strong magnetic fields, like those produced by the readout gradient, induce voltages in every loop both inside and outside the preamplifier. Even the use of miniature electronic components cannot adequately minimize the total surface in which currents can be induced within a standard preamplifier. Shielding can help reduce electric interference, but is less effective against magnetic interference. Materials, like mu-metal, normally used for this purpose, contain iron that effects field homogeneity, and are therefore also inappropriate for shielding.

Electrophysiological measurements as well as electrodes adapted for these measurements are described in WO 00/27279 A and US-A-4 640 290. General knowledge on measurement of electrical currents is disclosed by Iwansson et al. in "Handbook of sensors and actuators 7: Measuring current, voltage and power" 1999, page 110, ELSEVIER AMSTERDAM.

It is the object of the invention to provide new and improved methods and devices for measuring electrical currents in the presence of strong fields, as in magnetic field gradients.

This object is solved with methods and devices comprising the features of claims 1 and 6, respectively. Advantageous embodiments of the invention are defined in the dependent claims.

The invention is capable to be implemented in all applications for measuring electrical currents for simultaneous recording electrophysiological signals and measuring fNMR images. The electrical currents manipulated according to the invention can comprise dc or ac currents with a certain band width. With particular advantage, the invention is implemented with ac currents showing small high-frequency current changes.

The invention is applied with the simultaneous measurement of fNMR images and recording of electrophysiological potentials. The simultaneous measurement of fNMR images and recording of electrophysiological potentials of animals or human beings is a further subject of the invention.

With regard to the described example, the invention has particular advantages. The combination of fMRI with single and multiple electrode recordings in the anesthetized and behaving monkey is an ideal approach for systems neuroscience, as the two techniques are complementary, providing us with information on two different spatio-temporal scales. The electrodes have excellent spatio-temporal resolution but very poor coverage, while fMRI can yield important information on a larger spatio-temporal scale. Optical imaging of activity-dependent intrinsic signals has great spatio-temporal resolution (approximately 50 microns), and would appear to have both very good resolution and some coverage. Yet it is limited to the cerebral surface and it has proved very difficult to apply in the alert, behaving monkey, as it requires the replacement of dura mater with artificial, transparent materials, an operation that had extremely limited success.

Simultaneous recordings within the magnet have the additional advantage that provide us with rich information in experimental designs (such as those that can be applied in even-related fMRI for the study of perceptual multistability), in which average responses from different sessions, are insufficient for exploiting the information provided by the two techniques. In addition, simultaneous recordings of local field potential, single and multiple unit activity is of obvious importance for understanding the neural basis of different fMRI contrast mechanisms.

The invention presents the first simultaneous intracortical recordings of neural signals and fMRI responses. The inventors compared local field potentials (LFP), single- and multi-unit spiking activity with high spatio-temporal blood-oxygen-level-dependent (BOLD) fMRI responses from the visual cortex of monkeys. The largest magnitude changes were observed in LFPs, which at recording sites characterized by transient responses, were the only signal that significantly correlated with the hemodynamic response. Linear systems analysis on a trial-by-trial basis showed that the impulse response of the neuro-vascular system is animal- and site-specific, and that LFPs yield a better estimate of BOLD than the multiunit responses. These findings suggest that the BOLD contrast mechanism reflects the input and intra-cortical processing of a given area rather than its spiking output.

Further details of the invention are described in the following with reference to the attached drawings, which show:

Figure 0.1: Block diagram of the circuitry used to compensate for interference during the recording of electrophysiological signals within the magnet, simultaneously with the imaging sequences.
Figure 0.2: Microdrive and Electrode used in the "in-the-magnet" electrophysiological recordings.
Figure 0.3: Recoding and Imaging assortment (3 aus report)
Figure 0.4: Demonstration of the software denoising procedure.

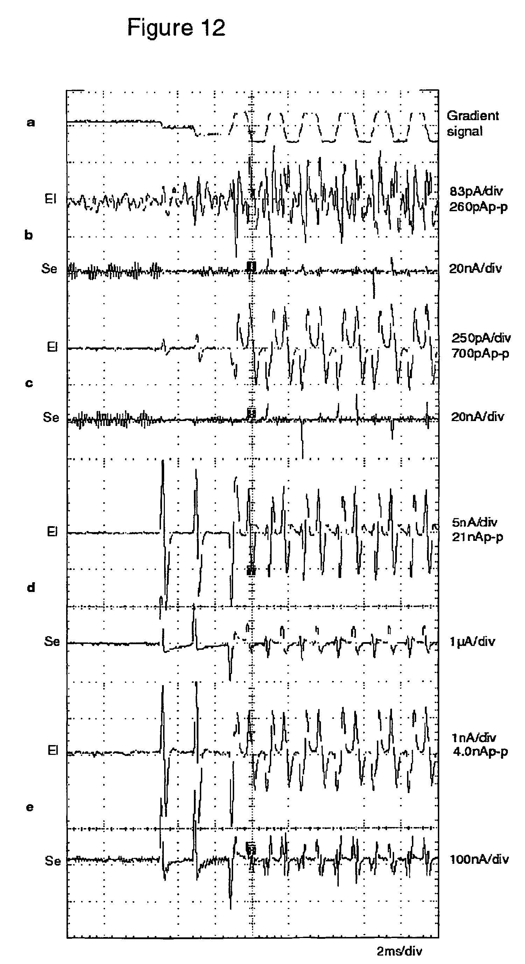

Figures 1 to 12: Illustrations of circuits for implementing various aspects of the invention.

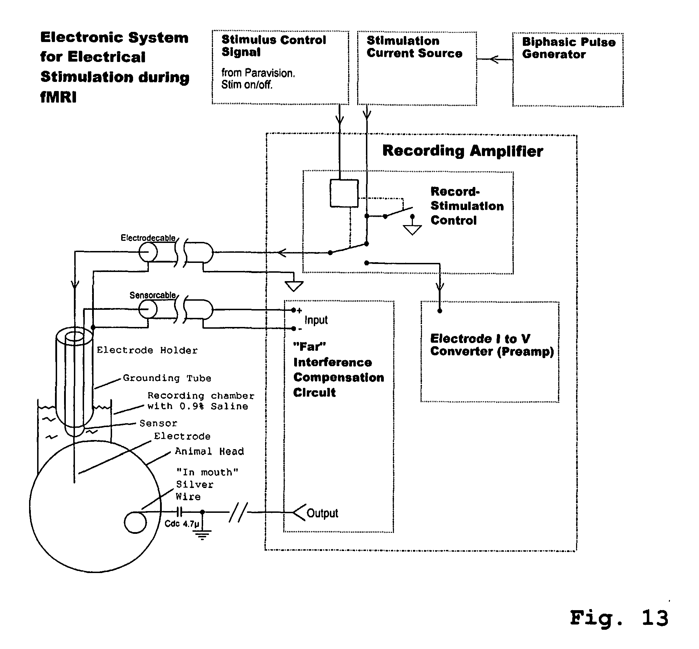

Figure 13: Electronic System for Electrical Stimulation during fMRI.
Figure 14: Principal circuit of the cable capacity compensation

Figure 15: Detailed circuit of the Cable Capacity Compensation Circuit.
Figure 16: Performance of the cable length compensation circuit.

## 1. Concepts and details of the invention

Low-noise, low-frequency voltage amplifiers, which are the ideal choice for the measurement of neural signals, possess the undesirable characteristic of rectifying high-frequency voltage signals coupled with the input signal (e.g. a square wave input signal will have a faster rise than fall time, which in high frequency tends to rectify the signal). This effect occurs at all stages of the IC including the operating voltage input or the output of the amplifier. In a 4.7T magnet amplifiers will immediately rectify the 200MHz excitation pulses. Our measurements with phantoms showed that the amplifiers quickly saturate and need several tens of milliseconds to recover after the signal has subsided. The addition high-frequency filters to every possible lead only lead to additional surfaces, or loops, in which the gradient fields induce voltages.

Moving the preamplifier outside of the magnet and the gradient tube does not solve the problem. The capacitance of the cable between the electrode and the amplifier (in our case 200pF for a 2m cable) and the impedance of the electrode itself act as a voltage divider that reduces the signal with increasing impedance and cable length.

To avoid signal loss in long cables, a method for measuring current, instead of voltage, was developed that is insensitive to cable length. It consists of measuring current flow coming from the electrode tip and converting it to voltage at the amplification stage (Figure 0.1). This method permits the measurement of signals over a cable length of 6m. Preamplifier, power amplifiers, operating elements, and interference compensation circuits are all packed in one single device, located outside the magnetic field.

The Recording Technique: The current measurement makes it possible that the transmission bandwidth from the electrode to the amplifier is not reduced by the long coaxial cable and that no energy is required for recharging the capacitance of the cable. In order to effectively measure the current signal it is important that no voltage drop occurs across the input of the preamplifier. For low frequencies this can be achieved by a classical current-to-voltage (C2V) converter that has a voltage drop of practically 0V. Yet, common C2V converters may show strong instability under our measurement conditions. In specific, the 600pF input capacitance (due cable length), together with the high-impedance feedback resistance, form a low-pass filter in the feedback circuit of the preamp, which reduces the phase margin (each amp defines the maximum phase shift that can be achieved without causing an oscillatory behavior at the amp's output) of the feedback at frequencies higher than the cut-off frequency so dramatically that the circuit becomes instable.

Thus, instead of the classical current-to-voltage converter a similar circuit using a voltage amplifier and a Proportional-Integral controller (PI controller) was used to ensure stability under the best possible signal bandwidth conditions. The tuning parameters (amplification and cut-off frequency) of the PI controller were set for the desired cable length by applying a test square-wave input signal across a 1Mohm resistor. They were adjusted to provide the fasted impulse response while yielding minimum overshoot. As in the classical circuit, the input current flows across a large resistance, and the voltage drop is equal to the output voltage of the converter. The output voltage of the circuit is adjusted such that the input voltage equals 0V. As a result, the input resistance of the converter is effectively 0 Ohm and the voltage at the output end of the resistor, with respect to ground, is equal to the voltage drop across the resistor. The voltage across the inputs of the converter is amplified using a low-noise DC voltage amplifier with a fixed gain. The input current of the amplifier is negligible.

The output voltage of the amplifier provides the actual-value signal for the PI controller. The set point is the ground potential. The PI controller adjusts the voltage on the output end of the resistor such that the output voltage of the amplifier equals 0V. In so doing, the input voltage of the converter is also adjusted to 0V. No current flows through the voltage amplifier, and, as a result, all of the current flows across the resistor. The voltage drop across the resistor can be tapped off from the output of the controller. Current flowing into the converter appears as a negative voltage at the output. The voltage at the output of the feedback resistor, i.e. the converted signal, can be loaded and used as the input signal to further stages.

Compensation of interference

The compensation of interference can best be described by distinguishing between the origin of the interfering signal, i.e. close to the tip of the electrode and far from the tip of the electrode. In our preparation we had to cope with two different sources of interference. One is introduced by the metal-to-electrolyte interface. The capacitance measured between the ground of the chamber and the animal's fluids is not zero. All currents induced into the "animal" will be capacitively coupled to the electrolyte within the chamber (we used 0.9% salt & agar in deuterium as ground; agar was used to avoid oscillations of the dura), and thus to the recording system's ground. This type of interference originates from a distance greater than that of the electrode-tip to the electrode ground. Interference caused by Eddy currents within the immediate vicinity of the electrode-tip cannot be compensated by measuring such an electrostatic coupling between animal-fluids and ground. This second type of interference is caused either by such Eddy currents or by imperfections in the rotational-symmetric shielding of the electrode wire, which will form at least a "partial" loop.

Compensation using a current sensor: Interference in the physiological signal due to capacitive coupling is actively compensated by a sensor built into the electrode assembly. It eliminates or greatly reduces all interference originating for sources more than a few centimeters away from the electrode. A simple electrical model of the animal is a conductor coupled to its environment through a number of capacitances. These capacitances are the metal-liquid transition zones around the animal. Firstly, the animal is coupled to the ground of the electrode through a very large capacitance. Secondly, the neuron (i.e. our current source), is coupled to the electrode through smaller capacitance (~500pF), which represents the resistance of the electrode. Finally, IV and ECG lines, eye irrigation lines, and SpO2 sensors were found to be strong sources of interference, by forming large loops sensitive to magnetic interference. Currents flowing because of such interference will pass through an effective electrode-to-animal-fluid capacitor to the electrode, because of the non-zero metal-to-fluid capacitance mentioned above (interference signals generated in the animal flow both across the electrode and the ground in accordance with their respective capacitances).

To compensate for this type of interference a third contact, namely the sensor electrode, is present near the electrode and has a lower capacitance to the animal than the ground of the electrode (Figure 0.2). The current between the sensor and the ground is measured, amplified and converted into a voltage. The voltage is inverted, amplified again in two steps with integrated output current limitation for the protection of the animal (10mA max, 2V max) and sent back to the animal through a junction electrode in the cavity of the mouth. This compensation secures that the current flowing back to the animal is just big enough to adjust the current into the sensor electrode to 0 A. As a result, there is no voltage change across the sensor capacitance and, consequently, no voltage change across the capacitances between ground and animal and between signal electrode and animal because these three capacitances are all parallel to each other. No interfering current flows into these capacitances and only the signal current from the neuron is detected by the electrode.

Compensation using a magnetic-field sensor: Interference induced close to the tip of the electrode, in the electrode holder or in the leads by the switching gradient coils cannot be compensated using the circuit described above. The magnitude of this interference is far too great and therefore requires a dedicated compensation circuit. To achieve this, the changing magnetic fields of the gradient coils are detected and the measured signal is used to generate a countercurrent. This is added to the input of the current-to-voltage converter in order to neutralize the interference. This method of compensation is effective against induction currents in the electrode holder and the cable which could not be completely avoided by a rotational-symmetric construction. In addition it is effective against induction currents due to the distance between the electrode tip and ground (eddy currents in the saline, in the recording chamber, and in the brain itself).

In specific, the magnetic-field sensor is composed of three small, identical, orthogonally-oriented coils positioned near the electrode. The voltages induced in the coils by the gradients serve as the inputs to the compensation circuit. These signals are amplified and passed through a further amplification stage where the gain can be adjusted with a precision potentiometer to a value between -1 and 1. The sum of the three independently adjusted signals serves as the reference of the current-to-voltage converter. The orthogonal orientation of the small coils and the adjustability of value and sign of the gain make it possible to simulate the induction voltage in a wire loop of any given diameter and orientation (vector addition). A virtual wire loop that has opposite sense of winding can be adjusted by the precision potentiometers in a way that the remaining loops caused by asymmetries in electrode holder and cable are effectively compensated. Figure 0.3 shows the assembly if electrophysiological and imaging devices used for simultaneous imaging and single or multiple unit data.

Software Denoising of Physiology Data

The above methods ensure a non-saturated, measurable signal which, however, is still contaminated by the gradient interference. The elimination of the rest interference was accomplished by applying two standard mathematical, dimensionality reduction methods, namely those of Principal and Independent Component Analysis (PCA and ICA respectively) techniques. The data are realigned to the segment onset pulse (signifying the beginning of collection of single K-Space segment), reshaped into an N by M matrix, where N is the number of segments and M is the number of data points acquired while digitizing the physiology signal. PC or IC analysis of such data and elimination of those components that best correlate with the directly recorded interference resulted in a "clean" signal as shown in Figure 0.4.

Methods

Figures 0.1 to 0.3 show further details of the recording hardware: (0.2, 0.3) Recordings were conducted with glass-coated platinum-iridium electrodes. Their holder consisted of three concentric, metallic cylinders, the innermost serving as the contact point for the electrode, the middle as the far-interference sensor, and the outermost as the amplifier ground (see Methods). The cylinders were insulated from each other by layers of PEEK. Electrode, sensor and ground were connected to the amplifier with two twisted, coaxial cables (middle panel). The electrode was positioned by manually turning a screw with a fine thread pitch. In addition, a three-coil magnetic-field sensor was mounted on the microdrive to compensate for near interference (see Methods). The RF coil used for imaging was placed around the chamber. (0.1) A block diagram of the recording and compensation circuitry. The animal can be conceived as being capacitively connected to any metal contact, including connections to ground ($C_g$), sensor ($C_s$), and electrode ($C_e$). Because of the finite animal-to-ground capacity, a fraction of the interference currents flow through the electrode. To compensate for such currents we used a sensor built into the electrode holder (Current Sensor). Interference originating in the vicinity of the electrode tip or within the electrode holder and the cables was compensated for by using three small, identical, orthogonally-oriented coils positioned near the electrode (Magnetic Field Sensor). C2V, Current to voltage converter; ADC, analog to digital converter, PIC, proportional integral controller.

This study involved 29 combined electrophysiological-fMRI experimental sessions in 10 healthy anesthetized monkeys weighing 6 to 9 kg. Surgery, Anesthesia for fMRI, and Visual Stimulation: Surgical procedures are implemented as described by Logothetis,N.K.et al in "*Nature Neuroscience*" 2, 555-562 (1999).

MRI Data Collection: Measurements were made on a vertical 4.7 T scanner with a 40 cm diameter bore (Biospec 47/40v, Bruker Medical Inc., Ettlingen, Germany). The system was equipped with a 50mT/m (180 micros rise time) actively shielded gradient coil (Bruker, B-GA 26) with an inner diameter of 26cm. A primate chair and a special transport system were designed and built for positioning the monkey within the magnet. According to one embodimend, customized small radiofrequency (RF) coils of 30-80 mm diameter were used (Figure 0.3) which were optimized for increased sensitivity over a given region of interest (ROI) such as a portion of the primary visual cortex or regions of areas V2, V3, V4 and V5 (MT). The coils were attached around the recording chamber and were used as transceivers. Alternatively, pick-up coils being arranged at the gradient coil cables are used. All images were acquired using a 96mmx96mm field of view (FOV). T1-weighted, high resolution (256x256, 0.5mm thickness) anatomical scans were obtained using the 3D-MDEFT pulse sequence (modified driven equilibrium Fourier transform, see Ugurbil,K. *et al.* Imaging at high magnetic fields: initial experiences at 4 T. *Magnetic Resonance Quarterly* 9, 259-277 (1993)), with an echo time (TE) of 4 msec, repetition time (TR) of 14.9 msec, flip angle (FA) of 20 deg, and 4 segments. In order to position the electrodes 45-slice anatomical scans were acquired with the FLASH (fast low-angle shot) sequence[48] with TE = 8.9 msec, TR = 2000 msec, FOV=96mmx96mm, and a 512x384 matrix, reconstructed to 512x512, slice thickness = 0.5mm. Multi-slice fMRI was carried out by using multi-shot (segmented) gradient-recalled echo planar imaging (GE-EPI)[49]. To examine the distribution of activation around the electrode, we initially collected volumes of 13 slices of 2 mm thickness, each with an FOV of 96mmx96mm on a 128x128 matrix. The acquisition parameters were: TE = 20 msec, TR = 750 msec, FA = 40 deg, EPI-zero-phase = 8.192 msec or 40% of phase-steps, pulse length (PL) = 3.0 msec, spectral width (SW) = 100kHz, line acquisition time = 1.28 msec, number of segments = 8, segment acquisition time (MRI readout window width) = 20.48 msec, repetition time between slices 50.36 msec, number of excitations per phase encode step (NEX) = 1. Finally, to study the time course of the BOLD signal and directly compare it with the neural responses, fMRI was carried out with GE-EPI with 1 or 8 interleaved segments, each segment acquired during a separate visual stimulation epoch using a 128x128matrix, TE/TR = 20/250 msec, FA = 20-25 deg, PL = 3.0 msec, SW = 100-150kHz, EPI-zero-phase = 20% of phase-steps, readout window width = 20.48 msec, and NEX = 1. The segments were acquired separately[20] and merged into one image by sorting all k-space lines according to their phase. In these segmented acquisitions, echo time shift (ETS) was used to minimize ghosting. The time of echo shift for a segment with the number j was calculated as (1+ j) * aqt / (number of EPI segments); aqt = matrix-size / (2 * spectral width), where aqt is the readout time for a single echo. In order to minimize the effects of inflow and of large drainage vessels we consistently used flip angles that were smaller than the computed Ernst angle by 10 degrees. In each session and for each monkey, an autoshim algorithm was used to tune the linear shim coils, followed by the manual tuning of the higher order shim coils. Shimming was typically performed with a hard pulse of 50 micro-seconds duration and a receiver bandwidth of 50kHz. Depending on the RF coil, either global or local volume shimming (FASTMAP[50]) was used. The line width (full-width-at-half-height) at the end of the shimming procedure ranged from 30 to 70Hz for the entire scanned volume. Together with the image acquisition both the plethysmogramm (detection of volume changes due to arterial pulsations, roughly corresponding to an ECG signal) and the flow signal of the ventilator were digitized at 250Hz and stored for later evaluation and removal of physiological artifacts from the BOLD time courses.

MRI Data Analysis: All data were analyzed off-line on Pentium computers running the Linux or NT operating system. Analysis was performed using our own software, MATLAB, and the MEDx 3.0 image-processing package (Sensor Systems, Sterling, VA). After normalization of the data, the linear trends were removed and the data were wavelet-filtered to remove temporal noise. Respiratory and cardiovascular artifacts were removed by examining the spectra of the recorded plethysmogramm and respiratory signal and creating appropriate filters. Respiration was kept constant in all experiments to 24 strokes per minute, corresponding to 0.417Hz (for a TR=250msec its sampling rate was 4Hz). To increase statistical significance, all images used for 3D brain reconstruction (voxel-size = 1x1x2 mm$^3$) were spatially fil-

tered (convolution filter, with full-width-at-half-height of 1.5mm, on a 3x3 kernel - roughly 95% of the filter is in the kernel). Both inter-group statistics (parametric paired t-test or F ratio) and correlation analysis were used to generate functional maps.

Electrophysiological Recording: All recording hardware, including electrodes, microdrives, signal conditioning and interference compensation devices, were developed in house (N.K.L., A.O. and M.A., in preparation). A plastic chamber (see Supplementary information) formed to fit the animal's skull precisely was implanted over the occipital pole during an aseptic surgical procedure. The skull inside the chamber was left intact. At the beginning of each experiment a 2mm trephination was performed through which the electrode was introduced into the brain. Electrodes were made of platinum-iridium ($Pt_{90}Ir_{10}$) wire etched with sodium cyanide (NaCN) solution and coated with glass (Corning glass 7570). The glass-coated tip was glued into a glass capillary tube (1.5mm outer diameter) with super glue. The wire extended 5-10mm beyond the end of the capillary tube and served as the contact point for the electrode. The electrode holder (Figure 0.2) consisted of three concentric, metallic cylinders (copper beryllium, CuBePbS or a bronze alloy, $CuSn_7PbZn$). The innermost cylinder served as the contact point for the electrode, the middle as far-interference sensor, and the outermost layer as the amplifier ground (see below). The cylinders were insulated from each other with layers of Polyetheretherketone. The concentric cylinders, in particular the outermost cylinder, offer the additional advantage of being a rotation-symmetric shield for the electrode, permitting optimal ground contact to the animal while avoiding any kind of loops susceptible to induction. The gap between electrode and electrode holder was sealed with silicon gel. Electrode, sensor and ground were connected to the amplifier with two twisted, coaxial cables. The outer conductor of both cables was connected to the ground cylinder of the electrode holder. The electrode holder was held firmly in a microdrive attached to the recording chamber via a holding ring. The positioning of the electrode was accomplished by manually turning a screw with a thread pitch of 700$\mu$m. In addition, a three-coil magnetic-field sensor (see below & Figure 0.1) was mounted on the microdrive. The joints between microdrive, holding ring and chamber were sealed with silicon gel. The chamber was filled with deuterium saline (0.9% NaCl in $D_2O$) to provide electrical contact between the ground cylinder and the animal. Deuterium saline was used instead of regular saline to avoid changing the RF coil's Q-factor and to permit optimal shimming. In addition, 0.6% agar was added to the saline to minimize oscillations in both the saline and the dura during the readout gradient alternations. At the beginning of each session the electrode was lowered into visual cortex and positioned such that both signal intensity and stability were maximized. Based on the anatomical MR images most recordings were obtained from granular and infragranular layers of V1.

Signal acquisition and conditioning: Several problems arise when one tries to apply conventional neurophysiology techniques in the context of MR imaging. Typically, a preamplifier is positioned near the head of the animal and is connected via a cable to a main amplifier some distance away. However, the strong magnetic field alternations required for the MR image formation incapacitate the preamplifier by inducing voltages in any existing loop in the circuit. Shielding reduces electrical interference, but it is ineffective against magnetic interference, while materials like mu-metals that attenuate magnetic interference contain iron, which affects field homogeneity. Furthermore, low-noise, low-frequency voltage amplifiers typically used in neurophysiology rectify high-frequency voltage signals coupled with the input signal, an effect that occurs at all stages of integrated circuits. In a 4.7T magnet, amplifiers immediately rectify the 200MHz RF excitation pulses, resulting in substantial disturbance of the output that only recovers several tens of milliseconds after the RF signal has subsided. Moving the conventional preamplifier outside of the gradient tube does not solve the problem, as the capacitance of the cable between the electrode and the preamplifier - in our case a cable of at least 2m - together with the electrode impedance, act as a voltage divider compromising the signal. To avoid signal loss due to increased cable length, we developed a method of measuring current instead of voltage that is insensitive to cable length. Specifically, current flow from the electrode tip was measured over an ultimate cable length of 6m and converted into voltage at the input stage of the amplifier, with preamplifiers, power amplifiers, operating elements and interference compensation circuits all packed into one single device located outside the magnetic field. Under our measurement conditions (approximately 600pF input capacitance due to cable length and high impedance of the feedback circuit of the preamplifier) common current-to-voltage converters are unstable. Thus, instead of a classical current-to-voltage converter, a voltage amplifier, a proportional-integral controller (PIC) and a 10MOhm resistor were used to ensure stability under the desired bandwidth conditions (Figure 0.1). The PIC regulates its output voltage so that the input voltage of the amplifier ultimately equals 0V. As a result the output voltage equals the voltage drop across the 10MOhm resistor and is therefore proportional to the measured input current. Furthermore, no signal loss occurs by charging the cable capacity. To obtain the fastest possible impulse response with the minimum possible overshoot for the desired cable length, the tuning parameters (amplification and cut-off frequency) of the PI controller were adjusted with a square-wave current.

Interference sources: Two types of interference had to be compensated for: interference originating from a distance greater than that from the electrode tip to the electrode ground (*far* interference), and interference originating from the immediate vicinity of the electrode tip (*near* interference).

Far Interference Compensation: Due to the metal-to-electrolyte interface the animal is capacitively connected to any metal contact (Figure 0.1), including connections to ground and electrode. Because the animal-to-ground capacity is finite, a fraction of the interference currents flowing toward the animal (e.g. currents through ECG or intravenous lines) will also flow through the electrode. To compensate for such currents we used a sensor built into the electrode holder. The current between the sensor and the ground was measured, amplified and converted into a voltage. The voltage was inverted, amplified again in two steps - integrated output current limitation for the protection of the animal (10mA max, 2V max) - and was sent back to the animal through a junction electrode in the cavity of the mouth. This compensation set the voltage of the mouth electrode so that the current flowing through the sensor electrode remained 0A. In other words, the mouth current equaled the sign-inverted interference current flowing to the animal. As the three capacitances (Figure 0.1) are parallel, setting the sensor current to zero forces all the other currents to zero as well, thereby eliminating the interference measured at the tip of the electrode and leaving the neural signal unaffected.

Near Interference Compensation: Interference originating and acting in the vicinity of the electrode tip or originating within the electrode holder and the cables cannot be detected by the sensor and compensated for by the circuit described above. The magnitude of this interference is sufficiently large to require a dedicated compensation circuit. Therefore, local magnetic field changes caused by the alternating field gradients during image acquisition were monitored by three small, identical, orthogonally-oriented coils positioned near the electrode, and the measured signal was added to the "ground" of the current-to-voltage converter in order to neutralize the interference. This method of compensation is effective against induction currents in the electrode holder and the cable, which could not be completely avoided by the rotational-symmetric construction described above. In addition this method is effective against induction currents due to the distance between the electrode tip and ground (possibly because of eddy currents in the saline in the recording chamber and in the brain). Briefly the operation principle of this circuit is as follows. The voltages induced in the coils serve as the inputs to the compensation circuit. These are amplified and passed through an additional amplification stage, where the gain can be adjusted with a precision potentiometer to a value between -1 and 1. The sum of the three independently adjusted signals serves as the reference (ground) for the current-to-voltage converter. The orthogonal orientation of the small coils and the adjustability of sign and value of the gain make it possible to simulate the induction voltage in a wire loop of any given diameter and orientation (vector addition). A virtual wire loop that has opposite sense of winding can be adjusted by the precision potentiometers in such a way that the remaining loops, caused by asymmetries in electrode holder and cable, are effectively compensated.

Neural Data Analysis: Signals were amplified by 3-30 mV/pA. In a conventional voltage measuring system using an electrode of 300kOhm impedance measured at 1kHz, this would amount to a total amplification of $10^4$-$10^5$. The bandwidth of the main amplifier was 50mHz to 3kHz (12dB/oct and 18dB/oct respectively) and the signal was digitized with 22.3kHz using a 16-bit AD converter, set to +/-10V input range. It was subsequently decimated by a factor of three to 7.43kHz. From the broad-band recordings three signals were extracted for analysis: (a) single unit trains of action potentials convolved with a Gaussian of fixed kernel (SD = 5ms) to yield spike density functions (SDF), (b) multiple unit activity (MUA) in the range of 300Hz to 3000Hz, and (c) local field potentials in the range of 10Hz to 130Hz. Band separation was accomplished either by filtering (10Hz-130Hz, 36db/oct and 300-3000Hz, 48db/oct, both with zero phase shift) or by selectively averaging the spectrogram (generated with a Hamming window of 250ms) of the signal across frequency. In the first case, multiple unit activity was rectified and low-pass filtered (150Hz, 24db/oct, zero phase shift) to obtain the envelope of the signal. Both separation methods yielded the same results. The same results could also be obtained using conventional electrophysiological techniques. Specifically, intracortical recordings were carried out with a 4x4 array of microfiber electrodes (quartz-$Pt_{90}W_{10}$; 80$\mu$m shaft diameter, spacing of 250$\mu$m center to center, impedance 250kOhm - 750kOhm, at 500Hz). The signal was amplified and filtered using BAK amplifiers (MMRS-1S).

Elimination of Residual Interference by Signal Processing:

The above interference reduction techniques ensured a non-saturated, measurable signal that, however, was still contaminated with a certain amount of gradient interference. The elimination of the residual interference was accomplished by applying a standard mathematical dimensionality reduction method, the principal component analysis (PCA) technique. The data were initially realigned to the slice-selection pulse (signifying the beginning of collection of an image of a single K-space segment for single- or multi-shot acquisitions, respectively), and subsequently reshaped into an N by M matrix, where N was the number of segments and M was the number of data points acquired while digitizing the physiology signal. PC analysis of such data and elimination of those principal components that best correlated with the directly recorded interference (obtained from the current-monitor output of the gradient amplifiers) resulted in a "clean" signal as shown in Figure 0.4. The quality of the denoising procedure was tested with artificial signals, and also by comparing the spectra of denoised signals with signals acquired in the same session (interleaved) without the gradient alternations. No difference in their spectra was found.

## 2. Measurement of current instead of voltage

If you measure voltage the input impedance of the preamplifier and the capacitance of the cable which connects the electrode with the preamplifier degrades the amplitude of the neuronal signal. On this account the preamplifier is normally mounted as close to the electrode, and thus the animal's head, in order to keep the cable capacitance low. In doing so the switching magnetic fields induce voltages in every loop both inside and outside the preamplifier. Even the use of miniature electronic components cannot adequately minimize the total surface in which voltages can be induced within a standard preamplifier. The appropriate shielding can help to reduce interference, however, while many options exist for shielding against electrical interference, very few options exist for shielding against magnetic interference. Moreover, the materials normally used for this purpose (e.g., mu metal) contain iron, which directly effects field homogeneity, thus effecting the fMRI signal and rendering them useless for such purposes.

Furthermore, a number of low-frequency voltage amplifiers possess the undesirable characteristic of rectifying high-frequency voltage signals coupled with the input signal. As a result, the rectified interference signal appears in the output signal. The effect occurs for coupling at the signal input stage, as well as at the operating voltage input and signal output stages of the amplifier. In the case of NMR, the amplifier rectifies the 200MHz excitation pulse of the NMR pulse sequence. Our measurements showed that under the conditions presented by our magnet such an amplifier is quickly fully saturated and needs several tens of milliseconds to recover after the signal has subsided. The addition of a high-frequency filter to every lead would only lead to additional surfaces, or loops, in which the gradient fields would induce voltages.

One solution to the problem of magnetic interference is to move the preamplifier outside of the magnet and the gradient tube. However, the capacitance of the cable between the electrode and the amplifier (in our case 200pF for a 2m cable) and the impedance of the electrode itself act as a voltage divider that reduces the signal with increasing impedance and cable length.

Two examples: 1) The electrode behaves like a capacitor with 200pF, which corresponds to an impedance of 800kOhm at 1kHz. In this case the cable capacitance of 200pF would lower the signal amplitude by a factor of two. The reduction would be independent of the frequency. 2) An electrode with 200kOhm. The reduction would be 20%.

In order to get around the problems associated with signal loss in long cables, a method for measuring current, instead of voltage, was developed that is insensitive to cable length. By measuring current flow coming from the electrode tip and converting it to voltage at the amplification stage, the problems associated with cable length can be avoided. We were able to increase the cable length to 6m, thus allowing us to combine all components (preamplifier, power amplifier and operating elements) in one compact device. The methods for compensating the interference of the gradient fields, discussed later, would be much more complicated and harder to operate in the case of spatially separated circuits.

### 3. Connection of the electrode

By using this principle of measurement the current flowing between the electrode tip, which is placed in the brain, and the ground connector, which is placed in the recording chamber, is measured. The ground connection is made of a bronze alloy (CuSn7PbZn, wstnr. 2.1090) and is placed annular around the electrode wire. The electrical contact to the skull is made with the aid of a mixture of agar (0.6%) with deuterium water and 0.9%NaCl which is filled in the recording chamber. The concentric ground connection serves also as a shield for the electrode wire against electric interference fields from outside the recording chamber. The electrode holder proceeds to two coaxial cables without any interruption of the shielding. A sort of cable is used which does not show the microphonic effect, that means it does not produce any electric currents if it is exposed to mechanical vibrations. Such vibrations occur strongly inside the magnet during the switching of the gradients.

At the bottom of the animal chair a feed-through for the electrode cable is mounted. This feed-trough is designed in order to short-circuit the shielding of the electrode cable with the animal chair for RF-currents but isolate it for low frequencies. An annular capacitor which spreads out from the cable shielding over the mounting plate of the animal chair serves for that purpose. This formation ensures a very low impedance at high frequencies because the annular capacitor does not include any connection wires which would have an inductivity and, as a consequence, a high impedance for RF-currents. This RF-short-circuit avoids the trespass of RF-interference into the magnet, which can disturb imaging, and, at the same time, prevents parts of the RF-excitation pulse from reaching the measuring amplifier via the electrode cable. One thing to think of would be to ground the animal by connecting the electrode ground to the grounded mounting plate. But as we will see later only the configuration described here allows recordings from more than one electrode.

It appeared that the remaining RF-pulse interference inside the electrode signal line was efficiently low, so no extra RF-filtering at the input of the measuring amplifier was necessary. Parallel to the electrode cable runs the sensor cable the role of which is discussed later.

The feed-through in the mounting plate for the sensor cable is designed in the same way like the one for the electrode cable.

But it turned out that these arrangements were not sufficient prevent RF-pulses from reaching the inside of the amplifier chassis, travelling on the outside of the shielding of the electrode cable and the sensor cable. Here the amplifiers rectify the RF-pulse resulting in an interference pulse in the electrode signal. Therefore, the feed-through type at the amplifier chassis was designed in the same way as that at the animal chair. By careful design of the annular capacitances on the surface of the front panel of the amplifier the problems with the RF-pulses were solved.

### 3.1 Ground management

It is necessary to isolate the electrode cable and the sensor cable from the front panel of the amplifier for low frequencies to avoid ground loops. After feeding the electrode cable through the front panel of the amplifier chassis a short coaxial cable follows which leads into a separate shielded box in which the current-to-voltage converter for the electrode signal is placed. Inside this box the shielding of the electrode cable is connected for the first time with the amplifier ground. This avoids that the electrode cable becomes a part of a ground loop.

The amplifier ground is connected with the chassis of the amplifier. The amplifier ground is not connected with protective earth. The "Far" Interference Compensation Circuit, which will described later, determines the potential of the amplifier ground with respect to the earthing potential.

Therefore, all outputs of the electrode signal and the control signals have to be referred to earth potential by using isolating amplifiers in order to be able to further process the signal with normally earthed instruments like oscilloscopes or plug-in cards for computers.

Consequently, two grounds exist in the amplifier which both are not connected to earth.

The first is the amplifier ground, which is connected to the ground of the electrode holder (it is represented by the simple three-cornered symbol in the circuit diagram).

The second is the ground of the outputs which are represented by the three-cornered symbol with the appendix "iso". The latter is normally set to earth potential by the connected instruments.

As a result of this ground management the chassis of the amplifier must not have contact with earth potential, and the isolation between the power supply system and the outputs of the power supplies have to be more effective in accordance with the regulations (European norm EN60742)

### 3.2 Connections to the Animal

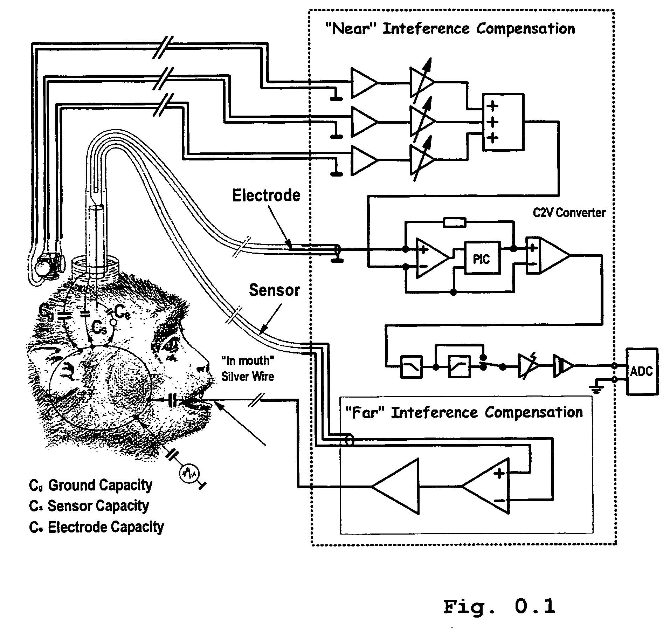

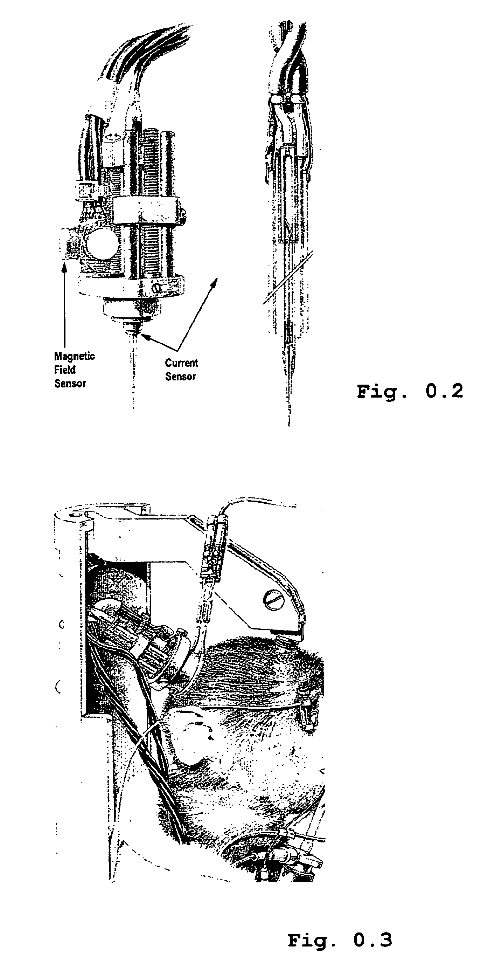

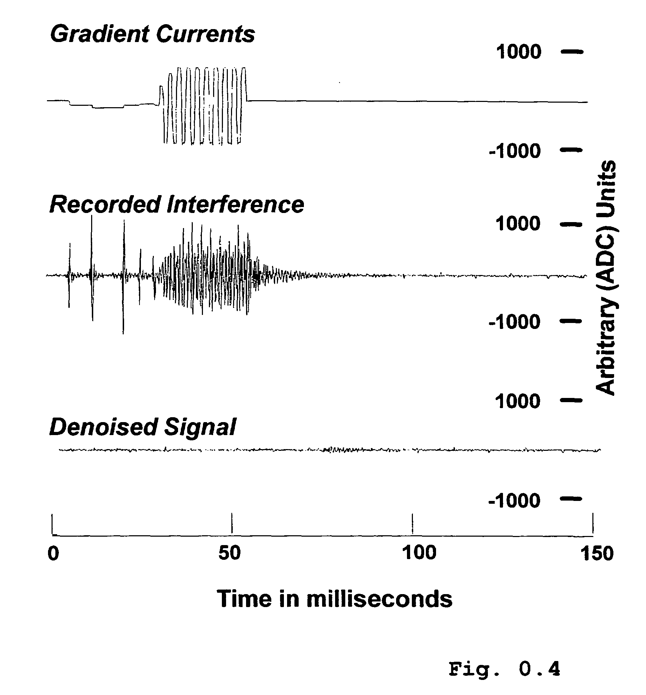

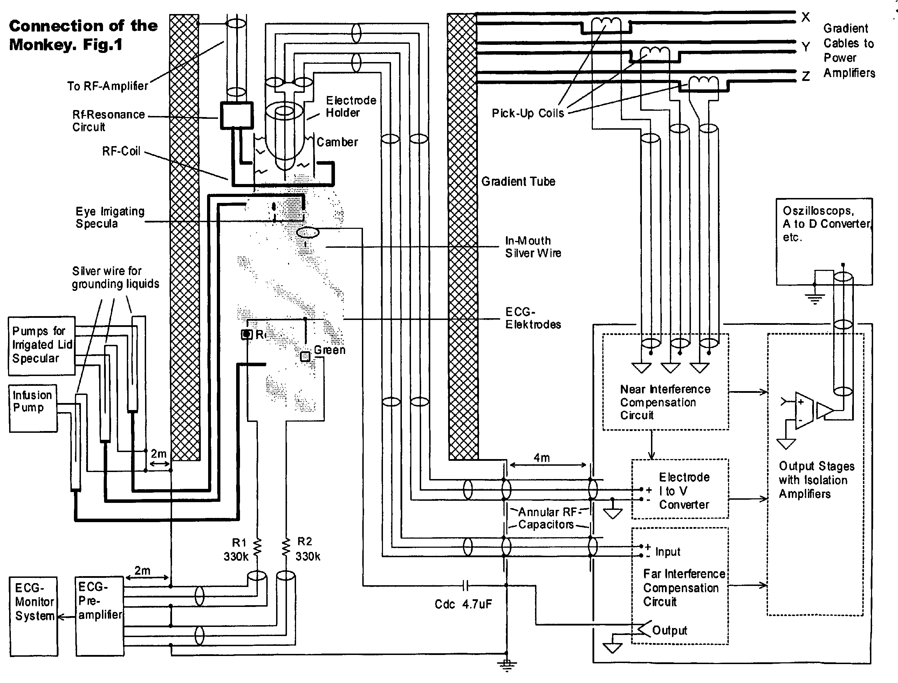

To be able to record inside the magnet while no imaging data is acquired, with all compensation circuits switched off, all interference sources were identified and appropriate countermeasures were taken. Some of these measures are not necessary if the far interference compensation is active. Figure 1 shows preferred connections.

### 3.2.1 RF-Coil

The RF-Coil is isolated from the animal. Thus, no low-frequency currents are able to flow to the animal. In addition the shielding of the RF-cable and the RF-resonance circuit is connected to the magnet and thus to earth potential.

### 3.2.2 Infusion line

The Ringer-Lactate-solution conducts at a frequency of 50Hz well enough to transmit interference currents to the animal which are coupled capacitively into the infusion line outside the magnet.

Especially the infusion pump and the infusion heater are sources for such interference signals. Interference currents which are coupled into the infusion line are drained away to earth potential by a thin silver wire (20cm long, 0.5mm diameter) inside the infusion line.

### 3.2.3 Eye Irrigating Specula

The eye lids of the animal are kept open with two specially designed plastic specula. In order to avoid any damage to the eyes from dehydration they are constantly irrigated with saline through small openings in the specula by an infusion pump. Like the infusion line the irrigating saline conducts interference currents towards the animal. Likewise, a silver wire earths interference currents.

### 3.2.4 Heating Pad

The body temperature is maintained with a heating pad. Distilled water is running through the heating pad forming a circular flow. The problems caused by the heating pad are minor compared to the ones originating from the infusion and the eye irrigation line. The heating pad is isolated from the animal and the water conducts much worse than the saline. Therefore, the heating pad is not presented in the diagram. Nevertheless, measures were taken to earth also the water inside the heating pad. Two clews of silver wire are placed inside the water hose near the feed-trough into the animal chair to achieve good contact to the water. The two silver wires are connected to earth potential.

### 3.2.5 ECG-Electrodes

The ECG-electrode connections are described in chapter 0.

### 3.2.6 SpO2-Sensor

Common SpO2-sensors produce electrical interference fields at a frequency of about 1kHz and contribute to a dynamic ground loop (see chapter 1.1) with their capacitance to the skin of the animal. The far interference compensation is able to remove both interference effects. An Sp02-sensor (Model 8600V, Nonin Medical Inc., Plymouth MN USA) operating with fibre-optic components was utilized that does not contain any conducting materials and, therefore, does not present any problems with electrical interference.

### 4 Record-Stimulation Control

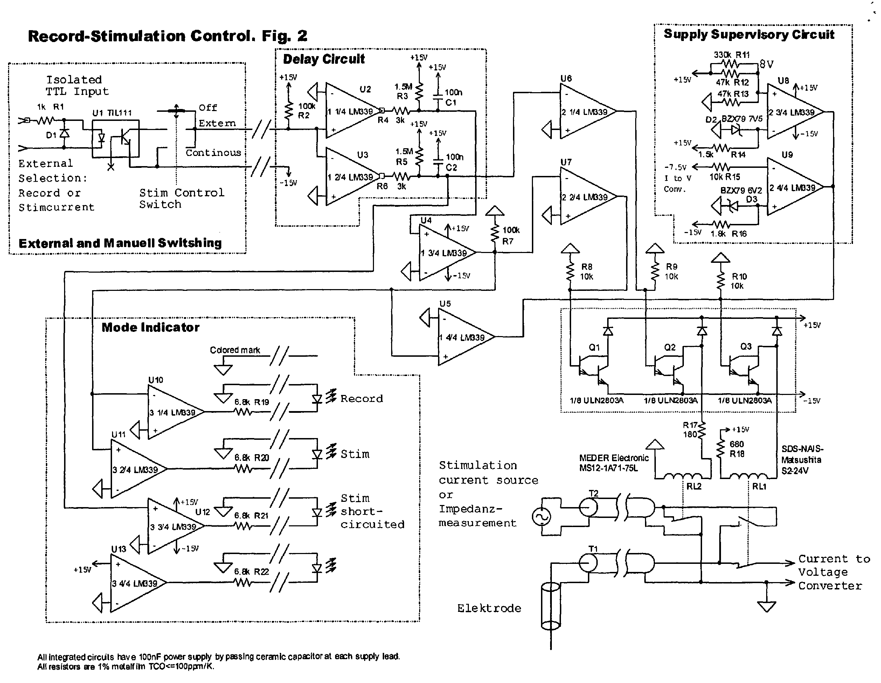

The record-stimulation control circuit (see figure 2) serves the following purposes :

1. The electrode can be disconnected from the current-to-voltage converter (i2uc) and connected to an external stimulation current source via a jack on the front panel.
2. The connection to the stim current source can be activated manually or externally through a TTL-compatible input.
3. The TTL-input has to be isolated from amplifier ground for reasons discussed in chapter 0.
4. The i2uc has to be automatically disconnected from the electrode if the instrument is switched off. This is an important point because the electronic input protection circuit for the i2uc is only working if the supply voltage is active.
5. The stim current source has to be always short-circuited whenever it is not connected to the electrode. When connecting the current source to the electrode it is important to firstly ensure the connection and only then remove the short-circuit. This procedure ensures that the current source is always loaded and that the output voltage can not saturate. Not following this procedure could result in the application of the entire compliance voltage to the electrode in the first moment with the effect of a high, severely harmful current in the brain. When the electrode is disconnected the short-circuit has to be closed first. Then the connection with the electrode is removed. The switching successions are given in the instructions of the manufacturer (BAK) of the stim current source used here.
6. The actual state of the switches is displayed by light emitting diodes (LED).

The isolated control input is accomplished with the opto-coupler TIL111 from Texas Instruments. R1 and D1 protect the LED of the opto-coupler against a voltage overload at the input. If the "Stim Control"-switch is set to "Extern" the transistor of the coupler operates with the resistor R2. In the position "Off" the electrode is permanently connected to the i2uc. In the position "Continuous" the electrode is permanently connected to the stim current source. In "Off" or "Continuous" mode the external control is deactivated.

The comparators U2 and U3 switch at the zero-crossing of the collector voltage. The open collector outputs with the networks R3, R4 and C1, and R5, R6, C2 respectively, generate a delay of about 100ms, depending on the direction of the zero-crossing of the collectors of the opto-coupler.

The comparators U4, U5 and U6 analyse the two delayed signals. The resulting signals control the driving circuits (Q2, Q3) for two relays which perform the switching successions claimed in point 5. The comparators U7 and U13 and the transistor Q1 are not used here and can serve for future circuits.

The relay RL1 (S2-24V from NAIS-Matsushita) is configured as a change-over switch and switches the electrode either to the i2uc or to the stim current source. In the current-free case of the relay coil the input of the i2uc is open (point 4). To ensure the drop-out of the relay during the fall of the supply voltages both supply voltages of the first op-amp (which has to be protected) of the i2uc are analysed. Comparator U8 with the resistors R11 to R14 and the zener diode D2 compares the positive supply voltage with the zener voltage and deactivates the relay RL1 as soon as the supply voltage drops from nominal +15V below +14V. Comparator U9 with R15, R16 and D3 compares the negative supply voltage of nominal -7.5V with the zener voltage and deactivates the relay RL1 as soon as the supply voltage increases to more than -6.2V.

The relay RL2 is closed in the current-free case so that eventually connected stim current sources are short-circuited if the instrument is switched off. This accomplishes an increased protection of the electrode.

The comparators U10, U11 and U12 analyse the control signals and control the LED's which display the actual state of the switches. A high quality relay for change-over switching for the electrode is utilized. It has a sufficiently high isolation resistance between the relay coil and its contacts. Furthermore, the contacts generate low noise. Both properties help to minimize interferences during recording.

5 Electrode Current-to-Voltage Converter

The current measurement makes it possible that the transmission bandwidth from the electrode to the amplifier is not reduced by the long coaxial cable and that no energy is required for recharging the capacitance of the cable. If in case of current measurement a voltage loss at the input of the amplifier can be avoided, as a consequence, no energy is lost in the capacitance of the cable and no current from the electrode is necessary to recharge that capacitance.

5.1 Protection of the input

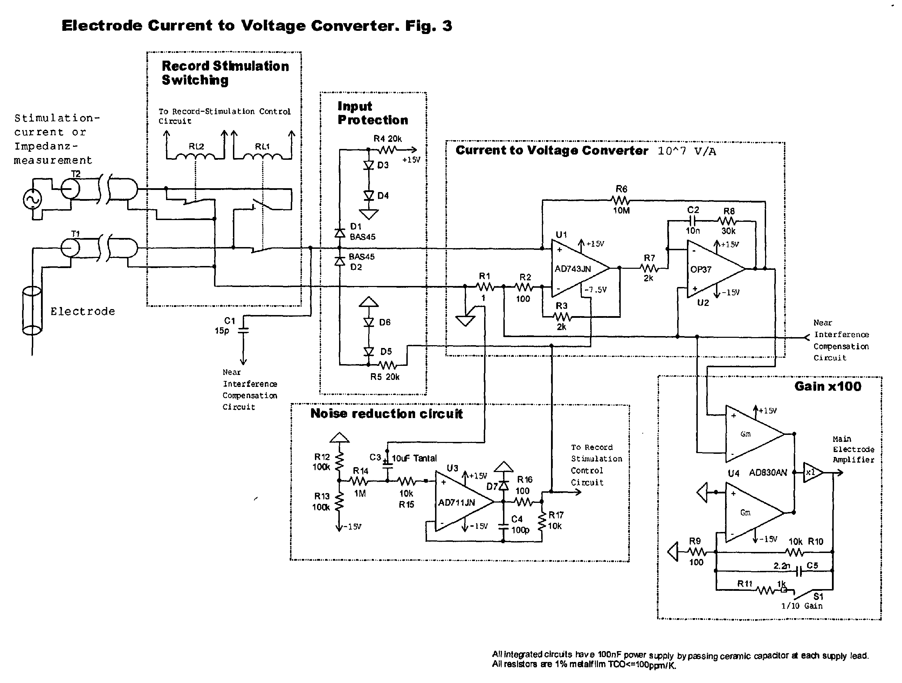

The electrode is connected with the operational amplifier (op-amp) U1 through the relay RL1 (figure 3). The function of RL1 is described in chapter 4. The op-amp AD743 must be protected from input voltages that are beyond its supply voltages. The diode network D1-D6 serves for that purpose. It limits the input voltage to a range from -2V to +2V. The maximum constant input current must not exceed 200mA. As a consequence, no special steps are necessary for the protection of the input of the amplifier during the handling of the device, e.g. changing of the electrodes. When the supply voltage drops below 2V, i.e. when the device is switched off, the circuit in chapter 4 takes care of the separation of the op-amp input from the electrode line. We used low-leakage diodes of the type BAS45 to avoid the injection of reverse current into the op-amp input during normal operation.

5.2 Circuit of the current-to-voltage converter

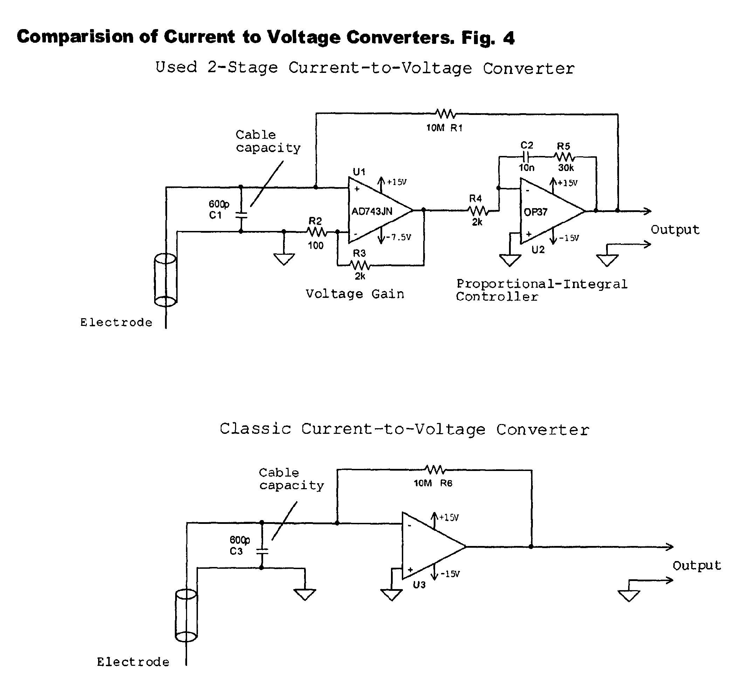

5.2.1 Comparison with the classical current-to-voltage converter Unlike the classical current-to-voltage converter with one op-amp the one described here is carried out with two stages. Figure 4 demonstrates both circuits. In either case the input current flows through a high-value resistor (R1 and R6). The voltage drop equals the output voltage of the converter. The voltage at the output end of the resistor is adjusted in a way so that the input voltage of the converter equals 0V and, as a consequence, the input resistance of the converter equals $0\Omega$ and the voltage at the output end of the resistor with respect to ground equals the voltage drop across the resistor. In both cases the input current is calculated from $I = -U_{output}/R$ (R=R1 or R6). Current flowing into the converter appears as a negative voltage at the output. For low frequencies this can be achieved by a classical current-to-voltage converter (see e.g. [Tietze, U. and Schenk, C., "Stromgesteuerte Spannungsquellen," Halbleiter-Schaltungstechnik 4 ed. 1978, pp. 240-241]) which has a voltage drop of practically 0V.

However, a capacitance at the input presents problems. For example, the 600pF input capacitance, due to the length of the cable, and the high-impedance feedback resistance form a low-pass filter in the feedback circuit (cut-off frequency here 27Hz) that reduces the phase margin of the feedback at frequencies higher than its cut-off frequency so dramatically that the circuit becomes instable.

Additionally, these effects depend on impedance of the electrode. A foreseeable flat frequency characteristic and a jump response without any overshoot was demanded. Therefore, instead of the classical current-to-voltage converter we developed a similar circuit using a voltage amplifier and a Proportional-Integral controller (PI controller) to ensure stability and the best possible signal bandwidth for a given cable length. The inserted voltage amplifier separates the high-resistive input of the i2uc from the input of the PI controller to ensure that the impedance of the electrode does not influence the control parameters.

Since the first stage amplifies only a voltage it can be optimised for minimal noise. This provides a better signal to noise ratio for the PI controller.

### 5.2.2 Voltage amplifier and noise

The i2uc (see figure 3) consists of a voltage amplifier with U1, a PI controller with U2 and the resistor R6. The voltage at the electrode line is amplified by a factor of 20 in the circuit consisting of U1, R1, R2 and R3. The op-amp AD743 from Analog Devices provides a very low-noise voltage amplification. In addition, the direct current fed into the electrode is negligible due to its FET input stage. This input stage also limits the current noise outwards the non-inverting input connection.

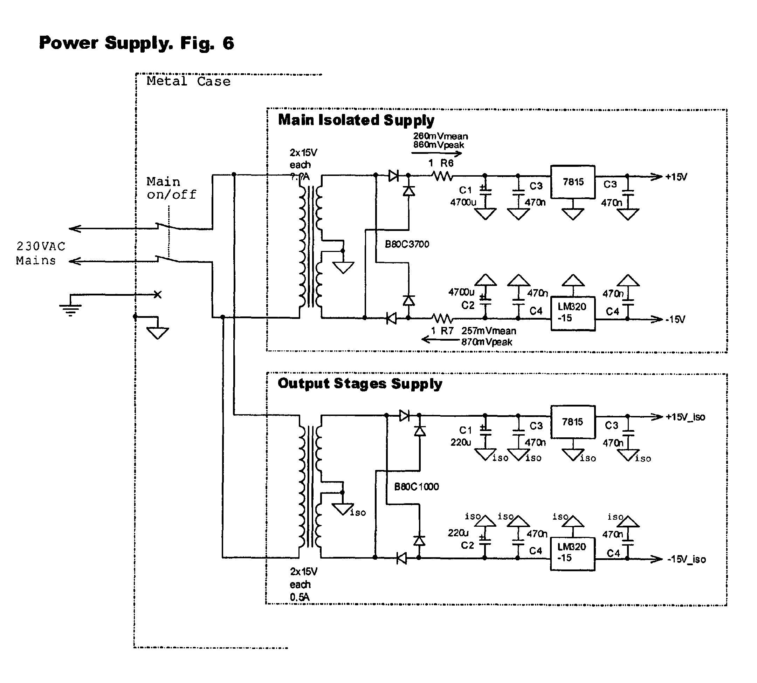

It turned out that the power supply rejection of the negative supply voltage of the AD743 was not sufficient to suppress the noise of the regular negative supply voltage to the desired level. That noise is generated by the voltage controller LM320-15 in the power supply unit (figure 6). To compensate that drawback the circuit designed with U3 generates a low-noise negative supply voltage of -7.5V for the AD743. These -7.5V are generated from the regular negative supply voltage by means of the resistors R12 and R13. R14 and C3 form a low-pass filter which blocks off the noise below the cut-off frequency of 0.016Hz. The op-amp U3 is wired as a unity-gain follower. The component parts R16, R17 and C4 provide the stable operation of the op-amp at the power supply bypassing capacitor of the AD743.

The noise of the device depends only on the noise in the preamplifier. This, in turn, depends on the length of the electrode cable and the internal resistance of the signal source. The lower the impedance of the cable or the internal resistance the higher the noise is. As a consequence, in our case even the impedance of the electrode influences the noise. For example, if a 6m cable is connected to the device and the bandwidth is set to DC - 3kHz with gain set to 1 the level of the output noise is $4mV_{rms}$ (measured with Tektronix TDS 420 with DC coupling after subtraction of any DC offset). The noise level of the input would be $4mV_{rms} / 1*10^9$ V/A = 4pA $_{rms}$. The thermal noise (Johnson noise) inherent to a resistor can be measured connecting a resistance greater than 100kOhm to the input. There is good correspondence between the measured (using the oscilloscope's high pass at AC setting as lower cut-off frequency, 10Hz) and theoretical values. In case of a 1MOhm resistor the noise current is 8pA $_{rms}$, with 100kOhm 19pA $_{rms}$. The measured values deviate more from the theoretical values with resistances smaller that 100kOhm. This is probably due to the fact that they are approaching the value of the non-ideal internal resistance of the current-voltage converter, which is 30kOhm at higher frequencies, see below. If the electrode is considered as an ohmic resistance between 100kOhm and 1MOhm, then the resolution of the amplifier is nearly as good as theoretically possible.

### 5.2.3 Proportional-Integral Controller

The following PI controller consists of the op-amp OP37 and the components R7, C2 and R8. The output voltage of the voltage amplifier referred to the voltage across the 1Ω-resistor R1 provides the control deviation. That voltage (not ground) represents the reference voltage for the i2uc. The design used here allows to feed a voltage change into the electrode cable. This possibility serves for the "Near" interference compensation (see chapter 10) to impress a compensating voltage into the cable. The same compensation circuit can couple a current into the electrode cable through the 15pF-capacitor C1. The 10MΩ-resistor R6 connects the output of the controller with the input of the voltage amplifier.

The PI controller adjusts the voltage at the output end of the resistor such that the output voltage of the voltage amplifier equals 0V. In so doing, the input voltage of the converter is also adjusted to 0V. No current flows into the voltage amplifier, and, as a result, all of the current flows across the resistor R6. The voltage drop across the resistor can be tapped off from the output end of the controller. Current flowing into the converter appears as a negative voltage at the output. The voltage at the output of the feedback resistor, i.e. the converted signal, can be loaded and used as the input signal for further stages.

The input current of the voltage amplifier results from $I_{input} = -U_{outController} / 10MΩ$. The proportional and integral tuning constants of the PI controller are set for a particular cable length. The components C2 and R8 which are determined experimentally define these constants. This is accomplished by feeding in a test square-wave signal into the circuit via the cable that will be used in the actual experiment. The current source are approximated with a square-wave voltage generator and a 1MΩ resistor. The tuning constants (amplification and cut-off frequency) are adjusted to provide the fasted impulse response while yielding minimum overshoot at the output end of the controller. A step-by-step description of the whole procedure can be found in [Tietze, U. and Schenk, C., "PI-Regler," Halbleiter-Schaltungstechnik 4 ed. 1978, pp. 673-676].

Generally, the control parameters in the current device are adjusted in dependence of the cable capacitance and the electrode impedance.

### 5.2.4 Gain 100 Stage and Prefilter

The following stage refers the output voltage of the PI controller to ground potential and amplifies it by a factor of 100. This is accomplished with U4, the high-speed video difference amplifier AD830 from Analog Devices. The difference amplifier contains two voltage-to-current converters (v2ic's), marked with Gm, and a voltage amplifier with gain 1. The input signal of the first v2ic is the output voltage of the controller. The feedback of the output voltage of U4 into the second v2ic with R9 and R10 yields a total amplification of 100. The switch S1 can shunt R10 and R11 and, in doing so, reduce the amplification to a tenth. The capacitor C5 reduces the amplification at high frequencies with a cut-off frequency of about 7.2kHz. This prefilter is inserted to avoid the saturation of the amplifier when high-frequency disturbing currents penetrate the electrode cable. An example for these disturbing currents is the modulation frequency of the pulse-width modulated gradient power amplifiers of our NMR system which is 81kHz. Through the gradient coils this frequency generates a disturbing current in the electrode cable. The overall gain of our device up to this stage results in $10^9$V/A.

6 Main Electrode Amplifier and Filter

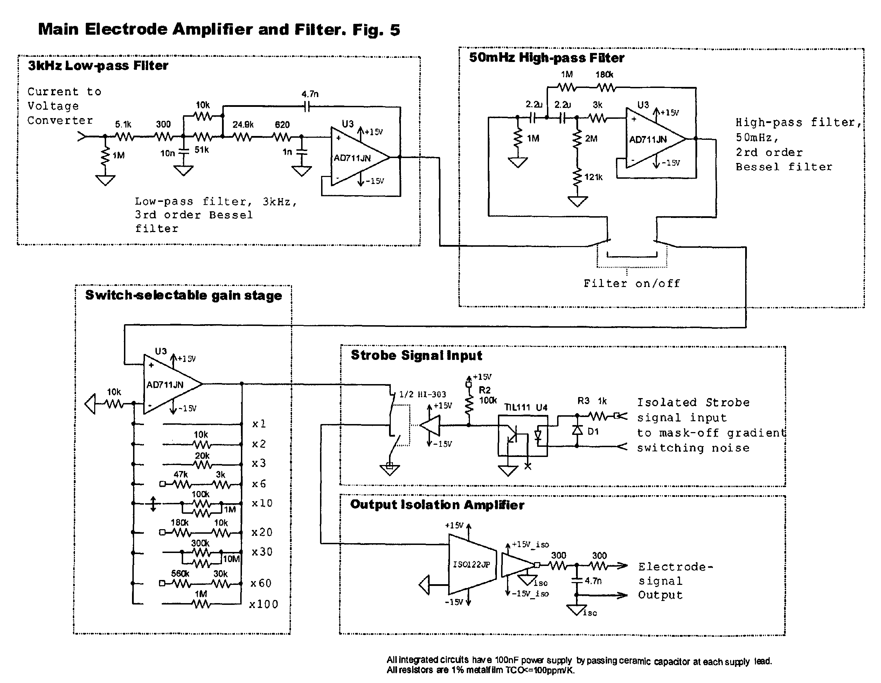

For our physiological recordings a bandwidth of 3kHz is sufficient. Consequently, a low-pass filter with a cut-off frequency of 3kHz follows the i2uc (figure 5). The filter is designed as a third order Bessel filter. The Bessel characteristic guarantees very good square-wave transmission properties with little overshoot. The calculation of the component values is described in the literature, e.g. [Tietze, U. and Schenk, C., "Aktive Filter," Halbleiter-Schaltungstechnik 4 ed. 1978, pp. 266-326.]. A high-pass filter follows which can be switched on as required. This filter is used only in cases in which a high DC current emerging from the electrode loads the dynamic range of the AD-converter too much which would require a reduction of the amplification. The lower cut-off frequency of 50mHz is low enough that no necessary information is lost in the recorded signal. The design of this filter is second order Bessel.

The next component in the circuit is an amplifier with switch-selectable gain (gain = 1, 2, 3, 6, 10, 20, 30, 60 or 100). The different values in small steps make it possible to best utilize the dynamic range of the AD-converter for the maximum resolution. A stepless adjustment with a potentiometer was rejected to ensure that the amplification does not drift or jump during physiological recordings. This is a very important point for the subsequent software processing of the data, namely the cleaning from the gradient noise.

The next stage allows the external muting of the output signal by means of a TTL signal applied to an isolated strobe signal input. This was done in preliminary experiments to mask off parts of the output signal which were strongly disturbed by the gradient switching noise. For that purpose we used the CMOS analog switch IC HI-303 from Harris (old model). As described in chapter 0, the control signal has to be galvanic separated. This is accomplished in the same way shown in chapter 4.

Finally, the output signal must be galvanic separated. Here the isolating amplifier ISO122 from Burr-Brown is used with the necessary high dynamic range (> +/-10V). This component does not function like an optocoupler. It transforms the input signal into a duty-cycle modulated signal and digitally transfers that one across the insulating barrier by means of small 1pF capacitors.

The output stage of the ISO122 converts the signal back into an analog voltage with the same amplitude as the input voltage. The passive low pass at the output end filters the carrier frequency of 500kHz originating from the digitalisation process. The output is short-circuit proof and has an output resistance of 600Ω. In spite of all the precautions taken, the isolating amplifier still has a noticeable noise level. This can result in the effect that at low gains of the main amplifier the noise of the isolating amplifier reaches the same magnitude as the i2uc. As a consequence, for sensitive recordings the gain of the main amplifier should be set at least to 3 in order to have the noise of the i2uc at a significant level at the output of the device.

7 Amplification factor

The description in 5 yields the following transfer equation: $V_{out} = -10^9$V/A $\cdot I_{in}$. Including the following voltage amplifier with gain set to 1 in the equation yields $1*10^9$ V/A = 1mV / 1pA. The gain of the entire device is adjusted at the level of the switch-selectable gain stage. Larger gains result in larger transfer factors (e.g., selected gain "x10" (see figure 5) →$1*10^{10}$ V/A = 10mV / 1pA). A 200kΩ electrode with tenfold amplification would result in a gain of $(1*10^{10}$ V/A) / 200kΩ = 50000.

A high-pass filter determines the lower cut-off frequency of the device. If the filter is off, the entire signal, including any DC component, will be amplified. The high-pass filter has $2^{nd}$ order Bessel characteristics and a -3dB cut-off frequency of 0.05Hz (50mHz).

The higher cut-off frequency is determined by a $3^{rd}$ order Bessel filter with a -3dB cut-off frequency of 3kHz.

(With the low-pass filter and the prefilter switched off the amplifier has a much higher bandwidth and a flat decrease in amplification at high frequencies. That course can be measured more detailed if required).

8 Power supply

As described in 0 the device possesses two different grounds. Therefore, two power transformers are necessary that generate two independent galvanic separated supply voltages, see figure 6. The protective earth is not connected inside the device which renders it necessary that the insulation of the transformers meet the EU standard EN60742. The first transformer generates a voltage of +/-15V for the main circuit with simple voltage regulator IC's. Two 1Ω resistors precede the regulators. They serve as safety fuse and offer the possibility to measure the supply current of the device.

The second transformer generates the isolated supply voltage for the output stages of the isolating amplifiers. This voltage amounts to +/-15V as well and is generated with the same type of voltage regulator IC's.

## 9 "Far" Interference Compensation Circuit

### 9.1 Dynamic Groundloops

The highest interference currents in the electrode line during the switching of the gradients arise as follows: The gradient fields induce a voltage in all conducting loops. This induced voltage depends linearly on the area inside the loop. The current flowing through the loop due to the induced voltage depends on the impedance one would receive when opening the loop at any place and measuring the impedance across both ends of the open loop. In doing so, one has to take into account the impedance not only for DC but also for the whole frequency range across which the neuronal signal shall be recorded. The steep slopes of the gradient currents include frequency portions from near DC up to 100kHz. The resulting interference signal meets exactly the frequency range which is required for the neuronal recordings. As a consequence, loops have to be taken into account that contain capacitances and, thus, form a loop only for alternating currents. We call these "dynamic groundloops".

In our case those loops which include the electrode cable cause the biggest problems. In such a loop the induced current flows across the ground connection inside the recording chamber. Since the contact resistance to the animal is not zero, a portion of the induced current flows into the electrode. This will be explained more detailed later. These circumstances make it indispensable that the capacitances between the animal and its surrounding are as low as possible, that all connections with the animal possess the highest possible impedance and that the areas which the connection lines form with the electrode line are as small as possible.

### 9.2 The ECG Preamplifier

It turned out that the application of our standard ECG device (ECG module M1001A; Controller Patient Model 84, M1178A; Monitor "Anaesthesia", Model M1094B; all Hewlett Packard) caused three major problems during combined electrophysiology and fMRI experiments.

1. The ECG electrodes with their connection lines to the ECG module and the electrode cable form a large groundloop inside the magnet in a way that the gradient fields induce interference currents in the electrode line. The ECG signal is acquired in order to monitor the depth of anaesthesia and to have a piece of information, completing the other acquired vital signals, about the physiological state of the animal. The heart rate is of the highest interest here and not the exact shape of the ECG signal. Neither is it necessary to receive a signal constantly. The monitoring between single scans in addition to the pulse rate derived from the SpO$_2$ signal is sufficient.

2. The ECG module injects a test current into the ECG electrode line to check the proper connection with the body. Additionally, the module can generate currents in order to actively compensate interference of the ECG signal. Both these properties are not desirable in our case because they interfere with the neuronal recordings.

3. Interference currents coupled capacitively into the ECG lines outside the magnet must not reach the animal.

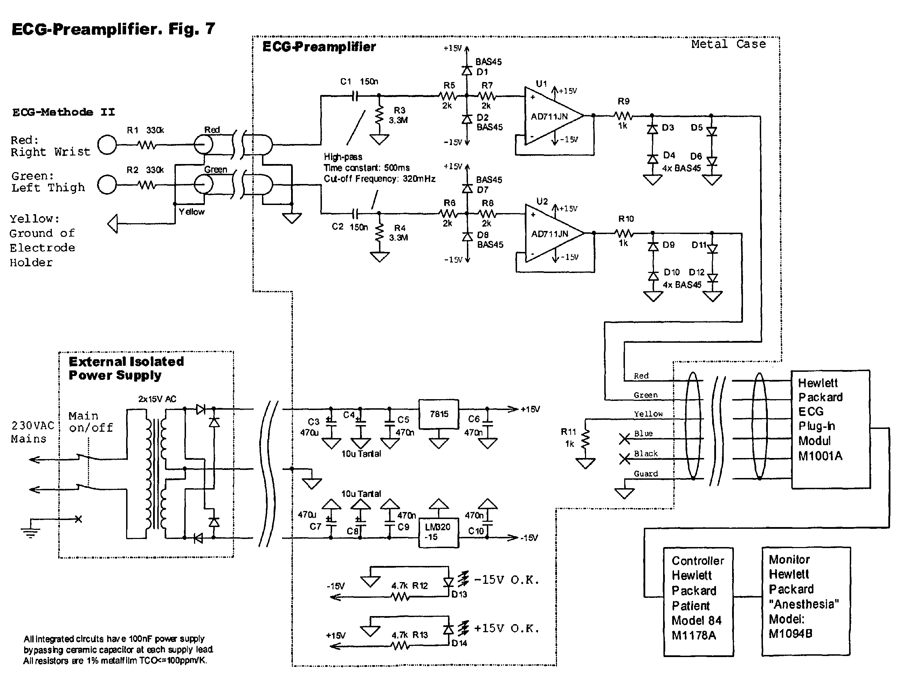

These problems led to the development of an ECG preamplifier that precedes the existing ECG module. This preamplifier is also utilized during neurophysiological recordings in anaesthetised animals without fMRI in our lab. Figure 1 shows the placement of the ECG electrodes and figure 7 displays the circuit of the preamplifier in detail.

Solutions to the problems:

1. The groundloops described above are rendered harmless with the aid of the resistors R1 and R2. Their values of 330kΩ each are high enough to minimize the induced currents to a level that they do not interfere with the signal in the electrode line. It is important that these resistors are placed between the animal and the shielded cable. The cable capacity of the shielded cable would neutralize the effect of R1 and R2 at high frequencies if the resistors were placed inside the preamplifier because in that case the induced currents would flow across the cable capacity. Even though the resistors R1 and R2 together with the 3m cable to the ECG preamplifier form a low pass for the ECG signal, the quality of the ECG signal is still sufficient to determine the heart rate properly.

2. Since only the determination of the heart rate is of interest here we limited the range of possible functions of the ECG system. We acquired the ECG signal only in recording mode Einthoven II. In that mode the potential difference between the right wrist (red electrode) and the left leg (green electrode) is measured. The yellow electrode at the left wrist normally serves as ground connection with the body. Here, however, the electrode holder serves as ground connection to the animal. When the gradients are not switching and the "far" interference compensation is not necessary the compensation circuit directly connects the animal ground to the magnet and thus to earth potential. The input to which the yellow electrode is normally connected is now connected to the magnet and can serve as a shielding for the two other ECG lines. The test currents that check the connection of the electrodes to the body are blocked from the animal by means of unity gain followers. As a result, there is no more feedback from the ECG module to the electrodes and, additionally, the interference compensation of the ECG module is deactivated.

3. The shielding of the ECG lines drains away interference currents to earth potential which could disturb the ECG signal. The chassis of the ECG preamplifier is constructed of metal and connected to the shielding. The connection lines between the magnet and the ECG preamplifier and the chassis of the preamplifier have to be installed carefully to prevent that they become a part of a groundloop which would affect the ECG signal. To avoid any other connection to earth potential except across the magnet an isolated current supply feeds the ECG preamplifier. The standard ECG module has a built-in potential separation like other medical equipment and, thus, no earth connection.

9.2.1 The Circuit of the ECG preamplifier

To avoid an overloading of following stages during a drift of the voltages the voltages from the ECG electrodes are fed into high-pass filters, consisting of C1, R3, C2 and R4 with a time constant of 500ms. The networks built of the components R5, R7, D1 and D2, and R6, R8, D7 and D8 respectively, protect the inputs of the following op-amps from overvoltage and overcurrent. The next stage with U1 and U2 (wired as unity gain buffers) serves as an impedance converter and stops any current flowing from the ECG module to the ECG electrodes. Here we used op-amps with very small input currents. The parts of the circuit carried out with R9 and D3-D6, and R10 and D9-D12 respectively, limit the output voltage of the preamplifier to 1.5V in order to protect the following stages from excessive overloading. The output voltages of the preamplifier feed the standard connections of the ECG module. The resistors R9, R10 and R11 by-pass the feature of the ECG system to check the placement of the electrodes. The power transformer with rectifier is placed in a separate chassis. Out of reasons mentioned before, its secondary winding is not grounded. Standard voltage regulator IC's generate two stabilized supply voltages of +15V and -15V. The LED's D13 and D14 display the state of operation.

9.3 An electronic model of the monkey

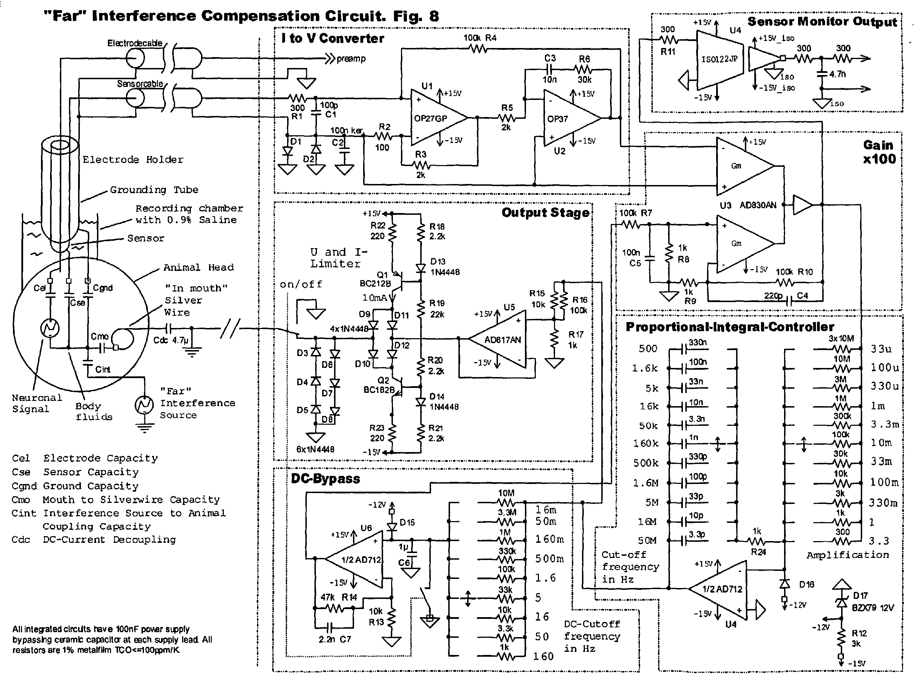

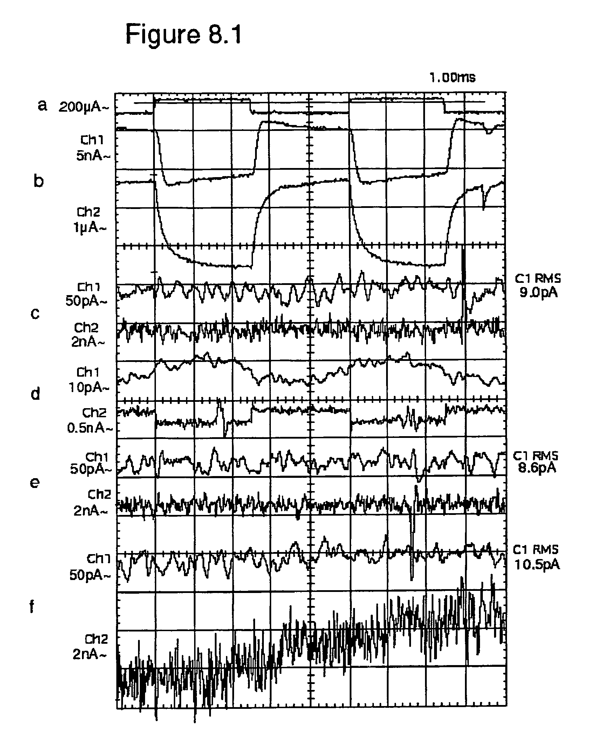

Figure 8 gives a simplified, equivalent circuit of the monkey. This model helps to clarify the way how interference currents appear in the electrode cable and how the compensation circuit counteracts them.

All metal-liquid transition zones can be characterized as capacitances. The ground connection around the electrode has a capacitance ($C_{gnd}$) towards the liquid inside the recording chamber which is in steady contact with body tissue. The tissue and the body liquids are considered ideally conductive.

In the annular design of the electrode holder the layer between electrode signal line and ground contact is the interference sensor that also posses a capacitance ($C_{se}$) towards the tissue. The capacitance of the electrode inside the brain ($C_{el}$) is by a factor of around 100 smaller than $C_{gnd}$ or $C_{se}$. $C_{el}$ is series-connected with a voltage source, namely the neuronal signal itself. Furthermore, we placed the mouth electrode (explained in 9.4) with the junction capacitance $C_{mo}$ into the cavity of the mouth. It is made of two 0.5mm silver wires wrapped around pieces of gauze soaked with saline. The "Far" interference source with the capacitance $C_{int}$ represents all interference sources that are coupled capacitively onto the animal from the outside, including the "dynamic groundloops" (see 9.1). The alternating currents fed into the animal across $C_{int}$ flow off across $C_{el}$, $C_{se}$, $C_{gnd}$ and $C_{mo}$. Since these capacitances are parallel to each other, the current is divided in accordance with their respective capacitances. The ground capacitance $C_{gnd}$ can only be increased up to a certain limit. Thus, a portion of the interference current always flows through the electrode ($C_{el}$).

9.4 Functional principle

The interference current into the electrode equals zero if the total interference current coupled through $C_{int}$ flows off through $C_{mo}$. In that case the effective interference current through the ground connection ($C_{gnd}$), the sensor contact ($C_{se}$) and the electrode ($C_{el}$) is zero. The current between the sensor and the ground is measured, amplified and converted into a voltage. The voltage is inverted, amplified and sent back to the animal through the junction electrode in the cavity of the mouth. This compensation ensures that the current flowing back to the animal is just big enough to adjust the current into the sensor electrode to 0A. As a result, there is no voltage change across the sensor capacitance and, consequently, no voltage change across the capacitances between ground and the animal and between the signal electrode and the animal because these three capacitances are all parallel to each other. No interfering current flows into these capacitances and only the signal current from the neurons is detected by the electrode. The compensation circuit does not influence the neuronal signal which is detected only by the electrode and not by the sensor.

It is highly import that this compensation method reacts very fast. The bandwidth of 3kHz of the neuronal recordings is not sufficient at all. The slopes of the interference originating from the switching gradient coils are so steep that even a small lag of the compensating signal can entail an interference pulse with its intensity proportional to the slope steepness and the lag time. Such a pulse, even though it is very short, contains all the lower frequencies in the range up to 3 kHz in its Fourier decomposition which would interfere with the data acquisition. Experimental data shows that with the optimal adjustment of the control parameters the compensation circuit is so fast that it can reduce the ripple of 81kHz originating from the PWM gradient power amplifiers to a fifth. (A performance test could be added here, e.g. recovery time after an interference pulse jump.)

For stationary DC the compensation circuit has to show completely different characteristics. The circuit does not have to function for DC because interferences from the gradient fields have no DC components. The function for DC is not even desirable for two major reasons:

    1. The DC resistance of the metal-liquid transition zones can strongly vary and reach high values. Not desirable contact voltages across the transition zones can occur. Thus, it is questionable if there exists a reasonable stationary situation in which the sensor current is zero.
    2. Provided that such a stationary situation exists a constant DC current could result from the different contact voltages. This could cause electrochemical processes in the transition zones which are not desirable at the tip of the electrode and inside the recording chamber, among others out of medical reasons.

To avoid these problems a large capacitor of $4.7\mu F$ was inserted in the line to the mouth electrode. In doing so, no more DC current can flow to the recording chamber. This capacitor, however, requires a completely different behaviour of the compensation circuit with respect to the frequency response. High frequencies require a high amplification and a small sensor current should result in a high counter voltage. At DC the amplification is ideally zero and the output voltage should be close to 0V and not depend on the sensor current.

9.5 The circuit

9.5.1 Sensor input

The electrode holder is composed of two concentric metallic cylinders (copper beryllium or lager bronze, see figure 8) to minimize induced voltages. The innermost cylinder serves as the contact point for the electrode wire conducting the neuronal signal. Around the electrode and the electrode contact is the sensor electrode that serves as the input to the compensation circuit. The two contacts are insulated from each other through a layer of TEKAPEEK (PolyEtherEtherKetone). The amplifier ground is concentric to both the electrode and the sensor and forms the outermost layer of the electrode holder. The ground as well as the sensor that measures the interference current into the animal stay constantly in contact with the liquid inside the recording chamber. Outside the chamber two separated coaxial cables with their shielding connected to the ground cylinder transmit the signals from the electrode and the sensor to further stages. Between those coaxial cables a voltage can be induced which makes it indispensable that they do not have any contact inside the measuring device. A connection would result in a groundloop and high interference currents in the shielding of the electrode cable. Consequently, the sensor input is designed floating. The diodes D1 and D2 protect the inverting sensor input from overvoltage. The capacitor C2 blocks high-frequency currents intruding from the shielding. The low pass (R1 and C1) filters high-frequency currents which might intrude through the sensor line.

9.5.2 Current-to-voltage converter

The current-to-voltage converter (i2uc) in the sensor circuit is very similar to that one described in 5. It has a local reference potential which is different from ground in order to disregard voltages induced between the shielding of the electrode cable and the sensor cable. Here the i2uc is designed with the bipolar op-amp U1 (OP27). The property of a low current noise is not needed here because the sensor impedance is by a factor of about 100 smaller than that of the electrode. For the same reason the resistor that accomplishes the current-to-voltage conversion has only 100k$\Omega$ (R4) instead of 10M$\Omega$. An interference current results in a signal of approximately the same magnitude at the output ends of both i2uc's. The control parameters of the PI controller are the same as in the electrode circuit. The next stage is again the difference amplifier AD830 (U3). It refers the output voltage of the i2uc to ground potential and amplifies it by a factor of 100. Also a prefilter is added (R9, R10, C4) with the same cut-off frequency as in the electrode amplifier. The components R7, R8 and C5 give the opportunity to add a DC voltage to the output voltage of the AD830 (see chapter 0). The transfer constant is 1:1. The output voltage of the AD830 feeds the isolating amplifier ISO122 where it is referred to the ground of the output stage (marked with "iso"). Thus, the magnitude of the remaining interference in the sensor can be monitored with an oscilloscope.

9.5.3 Proportional-Integral Controller

The output voltage of the i2uc determines the control deviation of the PI controller. The output voltage of the controller serves as actuating variable. It generates the counter voltage between electrode holder and the mouth electrode, as described in 0. The controller consists of the fast op-amp U4 (AD712), the resistor R24, 11 switchable resistors and 11 switchable capacitors. The resistors tune the proportional component of the controller in a range from $33\times10^{-6}$ to 3.3. The capacitors adjust the integrating component with integration frequencies ranging from 500Hz to 50MHz. The parts D15, D16, D17 and R12 avoid an output phase reversal of the op-amps U4 and U6 when their input common-mode voltage range is exceeded. The adjustment of the control parameters follows the method described in [B2]. The parameters depend on many influences, e.g. the impedance of the sensor and the ground contact, the conductivity of the mouth electrode and the capacity of the animal itself towards its surroundings. Normally, the adjustments are made only once in the beginning of an experiment.

9.5.4 DC-Bypass

In chapter 0 we pointed out the necessity of a special treatment of the stationary case. In the stationary case the actuating variable of the controller must stay as constant as possible and depend very little on the control deviation, i.e. the amplification is ideally zero. The previously discussed PI controller shows the opposite behaviour, its amplification tends towards infinite values. To solve that problem we inserted the DC-bypass. It replaces the whole regulated line section (including the animal) for frequencies below a few Hertz by a low pass. As a consequence, it restores the DC coupling between the actuating variable (the mouth electrode) and the controlled variable (the sensor current) which is missing due to the $4.7\mu F$ capacitor (see chapter 0). The first stage of the DC-bypass is the low pass already mentioned above. It consists of the capacitor C6 and 9 switchable resistors with its input connected with the output of the PI controller. The DC cut-off frequency can be adjusted in a range from 0.016Hz to 160Hz. The second stage (U6, C7, R13 and R14) amplifies the output voltage of the low pass by a factor of 6 and attenuates the amplification for frequencies higher than 1.5kHz. The output voltage of the second stage feeds the amplification stage of the i2uc with the AD830. Thus, this voltage is superimposed to the controlled variable delivered by the sensor. As a result, the amplification of the controller in the stationary case is decreased from infinite values to 1/6. During experiments it turned out that the adjustment of the DC cut-off frequency is uncritical. We constantly used a value of 5Hz.

9.5.5 The Power Amplifier

The power amplifier has to fulfil high requirements:

1. A high bandwidth is necessary (see 0).
2. The output signal has to reach its site of action quickly. The output resistance has to be small so that it does not form a low pass with the coupled capacities (e.g. between primary and secondary winding of the power transformer, isolating amplifiers, the capacitors at the bottom of the monkey chair (see chapter 0) or between the animal and the magnet) that has a too low cut-off frequency.
3. The power amplifier itself has to operate stably while loaded with these capacities.
4. In case of a failure like oscillation of the controller or a broken cable, the current flowing through the animal from the electrode holder to the mouth electrode must be limited to 10mA.
5. Additionally, the voltage that can possibly occur across the animal should be limited to a few volts for the protection of the animal.

The output voltage of the PI controller with a maximum of 15 V is stepped down to a maximum of 1.5V with the resistors R15, R16 and R17 (point 5). The op-amp U5 which is wired as a unity gain buffer buffers that voltage. We utilized the op-amp AD817 from Analog Devices which has a high bandwidth (point 1) and a special output stage that allows the op-amp to operate stably at infinitely high capacities (point 3). In this circuit the output resistance of the op-amp is practically zero (point 2). The design of the current limitation was a difficulty because in normal operating mode, i.e. with the current limitation inactive, it should have small output resistance even at high frequencies. The current limitation is accomplished with the diodes D9-D12 and two current sources that generate 10mA each flowing through these diodes. The current is divided up in two portions of 5mA in the branches D9/D10 and D11/D12. The input is situated between D11 and D12, the output between D9 and D10. The currents from the current sources with the transistors Q1 and Q2 flow in opposite directions referred to the diode network. Thus, no effective current flows into the input or the output and, in the unloaded case, the output voltage is equal to the input voltage. The output resistance is determined by the steepness of the diode characteristic at 5mA forward current and by the grade of equality of the forward characteristics. Consequently, we use the diode type 1N4448 which has tolerance values of the forward characteristic documented by the manufacturer.

If the output current exceeds 10mA the diode D11 blocks off any current and the output is supplied with 10mA through Q1 and D9. This limits the output current to 10mA. The same principle is valid for negative currents. The components R18-R21, D13 and D14 generate the reference voltages for the current sources from the supply voltage. The diodes D13 and D14 minimize the temperature dependence of the transistor current sources. Q1 with the resistor R22, and Q2 with R23 respectively, provide the 10mA currents with the aid of the reference voltages. The diode D3-D8 limit the output voltage in case of a current source failure to approximately 2V (point 5). The switch can deactivate the compensation circuit.

9.6 Performance test

In order to demonstrate the performance of the far interference compensation circuit we use a special approach. To make sure that we have only "far" interference to be compensated the test takes place outside the magnet. The electrode holder and the cables that are used in the experiments with animals are parts of the set-up. The test electrode has an impedance of 210kOhm. A saline bath simulates the animal. We use a rectangular saline bath with a surface area of 75mm*95mm and 50mm depth. In one corner a silver wire, similar to the mouth electrode described in chapter 0, is dipped in the saline. A small copper wire in the adjoined corner (about 50mm away from the silver wire) injects the interference current. The electrode holder is placed in a distance of 70mm to both wires. A voltage frequency generator and a 100kOhm resistor in series approximately represent a current source and generate the interference current. Additionally, a 10$\mu$F capacitor is inserted in series to avoid any DC currents and electrolysis in the saline. The ground of the generator is connected to the same contact of the recording amplifier where normally the magnet is connected, see Fig.1. The generator yields a rectangular signal with 200Hz and 7V p-p which results in about 70$\mu$A p-p interference current. As described above, a part of this current flows through the sensor and the electrode. The electrode amplifier is set to its lowest amplification 1*10^9V/A. The filter range is 50mHz to 3kHz. The amplification of the sensor monitor is 1*10^7V/A. The DC cut-off frequency is adjusted to 5Hz.

Figure 8.1 shows the electrode current on Ch1 and the sensor current on Ch2 under five different conditions (b to f). The corresponding current strength per division is labelled accordingly. The time base is 1ms per division. Diagram a shows the interference current signal.

In case b the far interference compensation circuit is almost deactivated by setting the control parameters proportional amplification and integrating frequency to the lowest possible values (33x10$^{-6}$ and 500Hz). The interference current is adjusted to the highest possible amplitude that does not saturate the two amplifiers. The resulting peak-to-peak interference current is 8nA in the electrode and 2.2$\mu$A in the sensor.

In the next condition c only the control parameters are changed to 0.033 and 500kHz. No interference can be seen in the electrode signal any more. Only the normal random noise originating from the electrode tip and from the i2uc remains with a magnitude of 9.0pArms.

In order to suppress the random noise and to increase the significance of the remaining interference the signal was averaged 50 times (case d) by means of an oscilloscope function. In the electrode signal only a 200Hz sine wave (6pAp-p) remains. Thus, the far interference circuit reduces the interference current by a factor of 8nA/6pA=1300. The sensor interference signal is reduced by a factor of 2.2$\mu$A/150pAp-p=15000.

In case e the interference contact is removed from the saline bath. No difference in the noise level can be seen compared to case c with the injected high interference current.

In case f both the mouth electrode and the interference wire were disconnected and the far interference circuit was switched off. Half a period (10ms) of the 50Hz mains interference of the lab environment appears in the sensor and electrode signal because the saline bath is not shielded appropriately. Even though the far interference circuit is inactive, the noise level of the electrode signal does not decrease, that is the far interference circuit in the normal operating mode does not increase the noise level of the electrode signal. Some spikes in the sensor signal in diagram c, d and e are related to the 50Hz interference and originate from the amplifier (the reason is not clear yet).

## 10 "Near" Interference Compensation Circuit

The "Far" Interference Compensation Circuit described above is able to suppress only interference currents which effect the sensor and the electrode likewise. Interference induced close to the tip of the electrode, in the electrode holder or in the leads by the switching gradient coils cannot be compensated using the circuit described above. The magnitude of this interference is too great and therefore requires a dedicated compensation circuit. This method of compensation is effective against local (near the electrode or the electrode cable) induction currents in the electrode holder and the cable which could not be completely avoided by a rotational-symmetric construction. In addition, we expect that this method is effective against induction currents due to the distance between the electrode tip and ground (eddy currents in the saline in the recording chamber and in the brain).

### 10.1 Functional Principle

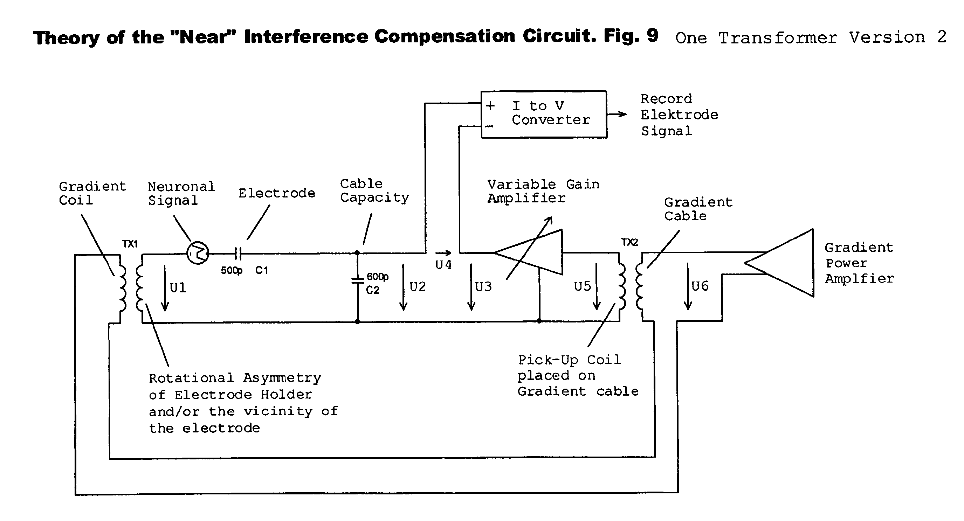

The simple model in figure 9 serves to point out the functional principle of this compensation method. (Figure with only one gradient coil in the model or the more complicated version with all 3 coils). The gradient coils induce voltages in the tissue surrounding the electrode and in the non-perfect rotational symmetry of the electrode holder. Firstly, we describe the conditions for one gradient coil which is simulated in the model by an ideal transformer TX1. Its primary winding represents the gradient coil itself. The secondary winding is series-connected to the electrode and the neuronal signal and generates the interference voltage U1. An ideal voltage source represents the neuronal signal while the capacity C1 (e.g. 500pF) is a model for the electrode. The capacity C2 (e.g. 600pF) simulates the electrode cable to the i2uc. The input voltage of the i2uc is U4 and equal to 0V in the stationary case. However, the input resistance is high for the fast rising slopes which can originate from the switching gradient fields. The variable gain amplifier can apply a compensating voltage U3 between the shielding of the cable and the inverting input of the i2uc. A pick-up coil which is placed around the supply cable of the gradient coil feeds its signal into the variable gain amplifier. Gradient cable and pick-up coil form a unit in the model and are represented by the ideal transformer TX2. Assuming ideal conditions, the voltages U1 and U5 are proportional to each other.

The example of a rectangular voltage jump U6 at the output of the gradient amplifier shall explain the principle of action of this compensation circuit. Such a voltage jump results in a linear rise of current inside the gradient coil and, as a consequence, in a linear rise of magnetic field strength. A voltage jump U1 is the obvious effect. The electrode capacity C1 and the cable capacity C2 step down U1 to the voltage U2 provided that the input resistance of the i2uc is high during the voltage jump. If the voltage U3 stays at 0V the voltage U2 drops across the input of the i2uc (U4). As a result, the i2uc drains away the charge in C2 and an interference pulse is generated. The variable gain amplifier can adjust the voltage U3 to the same magnitude as U2. Provided that the amplifier is adequately fast there is no more interference pulse at the input of the i2uc. This does not affect the neuronal signal because the voltages superimpose linearly. The variable gain amplifier must offer the opportunity to change sign of gain because the sense of winding of TX1 is not predictable. The gain of the variable gain amplifier is trimmed to minimize the magnitude of the interference.

For the more realistic case of three gradient coils the model has to be expanded as follows:

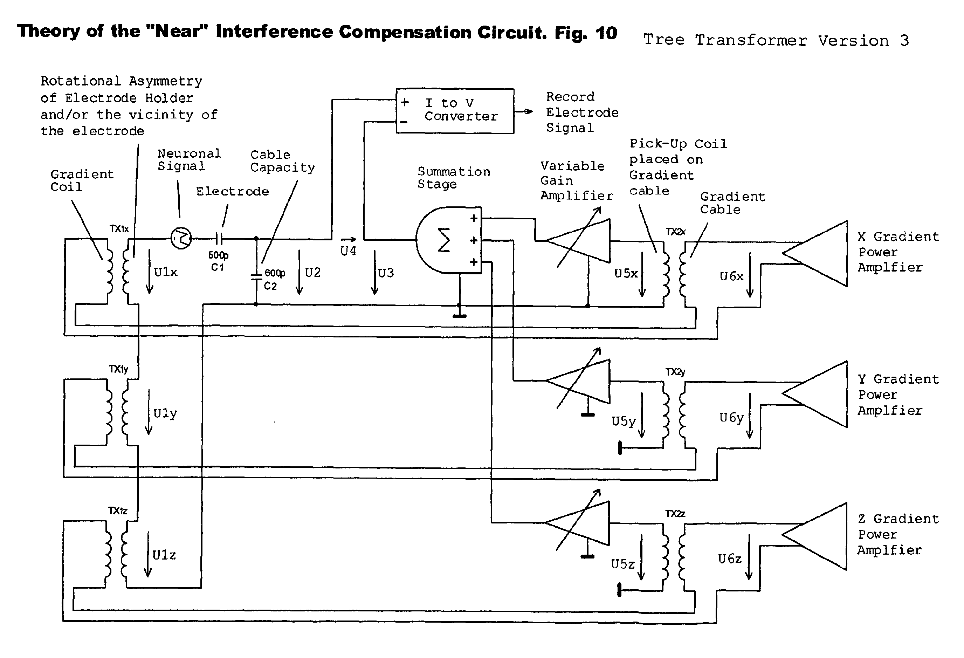

Two more gradient coils result in two more transformers like TX1, and two more like TX2 respectively (figure 10). The secondary windings of the three TX1 transformers (TX1$_x$, TX1$_y$, TX1$_z$) are series-connected and add their respective interference signal to the electrode. Each one of the three pick-up coils of the three TX2 transformers (TX2$_x$, TX2$_y$, TX2$_z$) has its own variable gain amplifier. The respective output voltages are added before they act upon the inverting input of the i2uc. The interferences originating from the three gradient coils and the three compensating voltages superimpose linearly and can be trimmed independently. The trim procedure of the three amplifiers is not trivial when the three gradient fields switch at about the same time because then it cannot be perceived from the interference signal which one of the amplifiers is still mismatched.

10.2 The Circuit

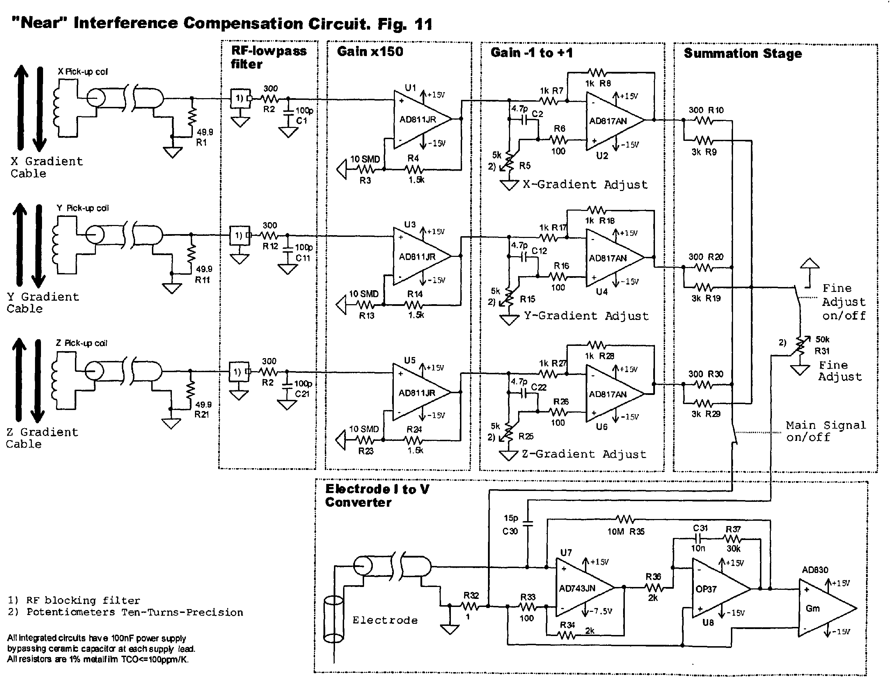

The circuit is the same for each one of the three pick-up coils. Therefore, we explain the circuit only for one pick-up coil (Figure 11). Each pick-up coil is connected to a 50Ω coaxial cable. Inside the device the 50Ω-resistor R1 terminates the cable. A purchased RF-blocking filter (40dB attenuation at 200MHz) is supplemented with a low-pass filter consisting of the components R2 and C1 (cut-off frequency 5MHz) in order to filter out potentially intruding high-frequency interferences. The next stage is a voltage amplifier with a gain of 150. Like in the "Far" interference compensation this circuit must be very fast (see 0). A wideband current-feedback operational amplifier is utilized to ensure the high rapidity of the circuit at this high amplification. The bandwidth of that kind of op-amp does not depend directly on the amplification like usually in op-amp's with voltage feedback. We utilize the AD811 from Analog Devices. The following stage can adjust the gain linearly between -1 and +1 by means of the high-precision potentiometer R5. This part of the circuit is described in literature, e.g. in [Tietze, U. and Schenk, C., "Bipolares Koeffizientenglied," Halbleiter-Schaltungstechnik 4 ed. 1978, pp. 194-195.] as bipolar coefficient element. It is accomplished with the op-amp AD817 from Analog Devices. It disposes of a high bandwidth and operates in the standard voltage feedback mode because no high amplifications are required. The resistors R7 and R8 and the potentiometer R5 together with the op-amp U2 trim the amplification between -1 and +1. The inductive behaviour of the wire-wound potentiometer hinders a high bandwidth of the circuit. The application of the capacitor C2 helps to lessen that disadvantage. The summation of the three output voltages, the attenuation of the signal and the low-resistance coupling into the electrode i2uc is accomplished with the resistors R10, R20, R30 and R32. R32 is a 1Ω resistor and is connected to ground. The other end of the resistor is connected to the respective output voltages through the resistors R10, R20 and R30. Thus, each output voltage is attenuated to 1/300 of the original value and the sum can be tapped off low-resistance across the resistor R32. This voltage corresponds to U3 in 0. The inverting input of the i2uc is connected to the summation point between R32 and R33. The coupling into the i2uc is described in 0.

The high amplification of the preamplifier and the subsequent attenuation yield:

1. a better signal-to-noise ratio for the variable amplifier.
2. a better signal-to-noise ratio for the propagation of the signal to the i2uc.
3. the possibility to feed the amplified signals from the pick-up coils into buffer amplifiers and monitor them with an oscilloscope.

Additionally, another summation signal of the output voltages of the pick-up coil amplifiers is generated, but with low attenuation. This is accomplished with the resistors R9, R19 and R29. The summation voltage can be diminished to 0V with the potentiometer R31. The output voltage of the potentiometer is coupled into the electrode cable through the small 15pF-capacitor C30. It often turned out that after minimization with R5, R15 and R25 the remaining interference could be reduced further with R31.

11 Performance test of both compensations

Here we present the performance of both interference compensation circuits during an experiment with an animal (session K00.7K1) and we show the effect of ground loops on the electrode interference current. The tip of an electrode with an impedance of 250kOhm is placed in the visual cortex of the brain of the animal. The fMRI-Sequence is, like in real experiments, an EPI one-shot sequence with two slices. The repetition time is TR=250ms. Figure 10 shows the electrode signal (labelled with El) and the sensor signal (labelled with Se) over a period of 20ms under four different conditions (b to e). In order to be able to align the appearance of interference effects with the switching of the gradient coils, the signals of the three current monitor outputs of the gradient power amplifiers were summed and displayed in diagram a. In case b the far and the near interference compensations are active and the peak-to-peak interference is adjusted to a minimum of 260pA by using all four potentiometers of the near interference compensation circuit. The amplifier gain here is set to 60*10^9V/A in order to take the most advantage of the ADC input range. In the following cases the amplification is reduced to ensure unsaturated signals. No spikes caused by the fMRI-RF-pulses appear. In the first 5ms the undisturbed neuronal signal can be seen because no gradient coil are switching during this time. The sensor signal is almost zero because the controller of the far interference circuit regulates this value to zero. The same is true for case c in which the near interference compensation circuit is inactive. The peak-to-peak interference increases to 700pA by a factor of 2.7. Additionally, in case d the far interference compensation circuit is switched off. This results in a strong increase of the interference in the electrode signal increases to 21nAp-p by a factor of 30. Now the interference currents can also be seen on the sensor signal. In this case the output signal of the far compensation circuit is connected to the amplifier ground (Figure 8). This configuration contains a big ground loop formed by the shielding of the electrode cable, the ground contact of the electrode holder, the head of the animal, the mouth electrode, the $4.7\mu$F capacitor and the cable to the far compensation circuit output which is connected to amplifier ground (Figure 1). This ground loop increases the electrode interference current as described in chapter 0. To demonstrate this effect a 1MΩ resistor in series with the mouth electrode discontinues the ground loop. The result is shown in diagram e. The interference current on the electrode and on the sensor signal decreases by a factor of 5. One could argue that it would be better to have this 1MΩ resistor constantly connected in order to reduce interference. But then the far interference compensation circuit would not operate fast enough because the 1MΩ resistor and the capacitance of the animal form a low-pass filter which would significantly reduce the best possible response time of the control loop.

## 12 Combined Electrical Stimulation and fMRI

### 12.1 Introduction

### 12.2 Electronic System

During electric stimulation the electrode remains connected to the main recording amplifier unit. The stimulation current is produced by means of an external current source which is connected to an additional input at the amplifier unit. Using the Record-Stimulation Control Unit the electrode can be easily and fast switched between the recording amplifier or the stimulation current source, either manually or by an external control signal.

The record-stimulation control circuit switches the electrode to the recording amplifier (Stim off) or to the external stimulation current source (Stim on) depending on the external control signal from the NMR acquisition program (ParaVision). The input signal is isolated by an opto-coupler. The unused current source (Stim off) gets short-circuited to avoid voltage build-up to the maximum compliance voltage (120V). Switching to "Stim on" this voltage would apply to the electrode instantly. This would damage the electrode and the cells as well. A delay circuit ensures the correct order of the switching of the relays to avoid an unloaded current source at any time.

A voltage-to-current converter with input-to-output isolation from BAK is used ("Biphasic Stimulus Isolator Model BSI-1") as the stimulation current source. The current source is modified to compensate stimcurrent loss in the capacity of the long cable to the electrode. The circuit is described in Chapter 0. As a necessity for the "Far Interference Compensation Circuit" to work properly the current output has to be floating.

The Far-Interference compensation circuit, originally designed to minimize interference during recording generated by the switching gradients, is always active and minimizes all gradient-induced currents to less than 100nA.

A waveform generator with voltage output (BAK Biphasic Pulse Generator Model: BPG-Z) generates the desired biphasic voltage pulses which drive the voltage-to-current converter.

Some of the waveform parameters of the stimulation current remained unchanged in all stimulation experiments:

1. Polarity of first pulse of the biphasic pulse: The (technical) current flows outwards the electrode.
2. Polarity of second pulse of the biphasic pulse: The (technical) current flows inwards the electrode.
3. Pulse duration: $300\mu$s for the first and the second pulse.
4. Symmetrical current strength: Current strength of the first pulse is equal to the strength of the second pulse, but with opposite polarity.

These parameters were varied:

1. The frequency of the appearance of the bipolar pulses.
2. Current strength.

### 12.3 Cable Capacity Compensation

### 12.3.1. Circuit Principals

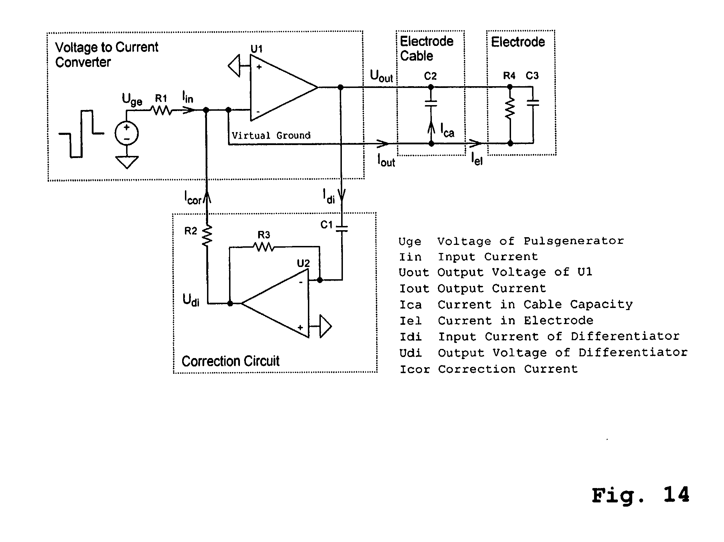

Figure 14 shows the principal circuit of the cable capacity compensation. The biphasic voltage pulses are isolated by an opto-coupler. Its output voltage is marked by $U_{ge}$. $U_{ge}$ drives the voltage-to-current converter which drives the electrode. The classic current source circuit with an inverting operational amplifier is used. $U_{ge}$ is applied to the resistor R1 which generates the desired stimulation current.

The other terminal of the resistor is set to virtual ground potential by the op-amp U1. Thus, the current $I_{in}$ flowing through R1 is equal to the output current $I_{out}$ which again is equal to the current $I_{el}$ flowing through the electrode.

But with a long cable connected to the electrode the cable capacity must be taken into account. The output current $I_{out}$ then flows through the electrode cable with the capacity C2 to the electrode, which is represented by the resistor R4 and the capacitor C3 in parallel. The output current splits into two portions, $I_{ca}$ that charges the cable capacity and $I_{el}$ that flows through the electrode. Note that it is not possible to directly measure the current $I_{ca}$.

Thus, the current $I_{ca}$ reduces the desired current $I_{out}$ to the electrode. The principle of the correction circuit is to analyse the voltage applied to the cable, then to calculate the current which is required to charge the cable capacity and, finally, to increase the output current by that amount of current. As a consequence, the loss in the cable capacity is compensated.

That correction current $I_{cor}$ is injected into the virtual ground like the input current $I_{in}$.

The current charging the cable capacity can be calculated by differentiating the voltage across the cable. The voltage across the cable can be taped off low-resistance from the output of the op-amp ($U_{out}$) referred to ground. $U_{out}$ serves as the input voltage of a differentiating circuit which is designed with the op-amp U2, the capacitor C1 and the resistor R3. Its output voltage is named $U_{di}$. R2 converts this voltage into the correction current $I_{cor}$. The values of the components in the correction circuit R2, R3 and C1 can be deduced as follows :

The values are calculated to achieve the equality of the input current and the current through the electrode: $I_{in} = I_{el}$ (1). Assuming ideal op-amps U1 and U2, the net current from the inverting input of U1 into the virtual has to be 0A: $I_{in}+I_{cor}-I_{ca}-I_{el}=0$ (2). Inserting (1) in (2) we obtain: $I_{cor} = I_{ca}$ (3). The current through a capacitor is in general proportional to the temporal derivative of the voltage across its terminals. This yields for the cable capacity:

$$-I_{ca} = \frac{dU_{out}}{dt}C_2 \quad (4) .$$ The voltage $U_{out}$ also

applies to the capacitor C1 because the other terminal of C1 is connected to virtual ground potential through the op-amp U2. The current through C1 can also be calculated from :

$$I_{di} = \frac{dU_{out}}{dt}C_1 \quad (5) .$$

This current produces a voltage drop across the resistor R3 which is equal to $U_{di} = -I_{di}R_3$ (6). $U_{di}$ across R2 generates the correction current

$$I_{cor} = \frac{U_{di}}{R_2} \quad (7) .$$ Inserting (5) and (6) in (7) we receive : $$I_{cor} = -\frac{dU_{out}}{dt}C_1R_3 \Big/ R_2 \quad (8) .$$ In-

serting (4) in (8) yields : $$I_{cor} = \frac{C_1R_3}{C_2R_2}I_{ca} \quad (9) .$$

As a result $$\frac{C_1R_3}{C_2R_2}=1 \quad (10)$$ has to be valid to solve (3). With (10) the parts of the correction circuit R2, R3 and C1 can be calculated with a given cable capacity C1. In practice one resistor is variable and the circuit is trimmed to the cable length with this potentiometer.

Note that the settings do neither depend on the electrode impedance, R4 and C3, nor on the shape of the stimulation pulse. The setting of the compensation circuit only depends on the cable length and no further adjustments are necessary in the course of a stimulation experiment.

12.3.2 Circuit Details

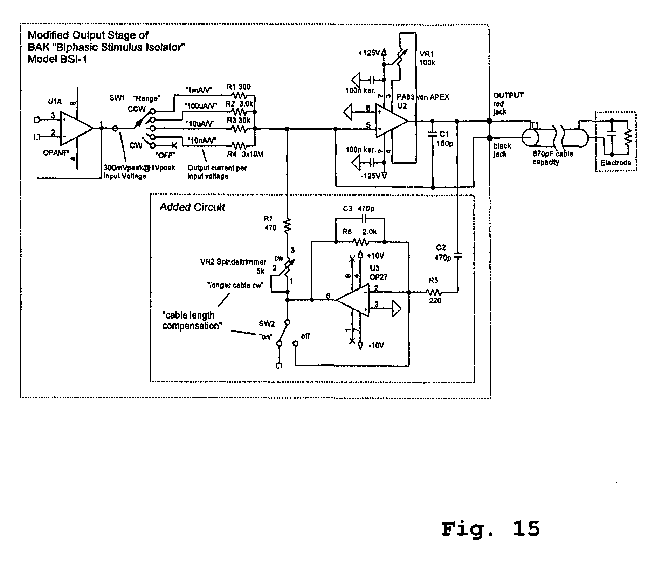

Figure 15 shows the output section of the voltage-to-current converter (u2ic) of the BAK "Biphasic Stimulus Isolator" Model BSI-1. The high output voltage op-amp PA83 from APEX is used to provide a compliance voltage of more than 100V which is, for example, required in order to drive a 1MΩ resistor with a current of 100μA. An additional circuit which offers the opportunity to monitor the output voltage of the current source by means of a regularly earthed oscilloscope isolates the output voltage and transfers it to the ground of the input. This circuit is not shown here.

Based on our experience with electrodes up to 330kΩ, measured with 1kHz and 20nApeak, and stimulation currents up to 450μA, the voltage never exceeded 10V. The impedance of the electrode is highly non-linear. It declines strongly with increasing current. The impedance of a 330kΩ electrode dropped to 30kΩ driven with approximately 300μA which limited the voltage to 10V (=30kΩ x 300μA).

The "isolated side" of the isolator is powered by two bipolar supply voltages of +/-125V and +/-10V, which are supplied by two switching power supply modules.

The input voltage of the isolator is transferred to the isolated side with opto-couplers. The output voltage of U1A, see figure ??, is proportional to the input voltage with a factor of 300mV per 1V input voltage. U1A drives the input of the u2ic. Several parts of the original BAK circuit were removed or changed. A classical inverting u2ic circuit with one op-amp U2 is used (Tietze, U. and Schenk, C., "Stromquellen für erdfreie Verbraucher," Halbleiter-Schaltungstechnik 4 ed. 1978, pp. 242-243). The output voltage of U1A is connected through the "Range" switch SW1 to one of the resistors R1, R2, R3 or R4 which are all connected to the virtual ground provided by U2 at its inverting input and generates the output current.

The output terminals are used interchanged compared to the original BAK configuration. The low-resistive output terminal (red jack) which is connected to the output of U2 now serves as the output ground and is connected to the shielding of the electrode cable. Therefore, the sign of the transfer factor of the isolator now is negative (positive input voltage provides negative output current).

C1 ensures the stability of the u2ic if only a short cable is used to connect the electrode.

As described in 0, a correction circuit had to be added to the u2ic. However, the circuit presented there does not work with realistic op-amps. The circuit in figure 2 would act like an oscillator because U1 and U2 are connected in a circle. At a certain frequency the total phase shift exceeds 180degrees while the total amplification is still high. To prevent instabilities in the real circuit (see figure 15) the amplification at high frequencies was lowered without increasing the phase shift. The fast op-amp U3 (the OP27) is utilized in the correction circuit in order to move the phase shift effects to the highest possible frequency. The parts C2 and R6 provide regular differentiating operation of the correction circuit at normal frequencies. At high frequencies the components C3 and R5 are more dominant and the circuit tends to behave like a classical integrator with the desired behaviour with regard to phase shift and amplification. The values of C3 and R5 were determined experimentally in order to provide circuit stability and not to degrade the usual cable capacity compensation speed.

In 0 the remaining speed of the circuit is demonstrated with experimental data.

In theory the values of the parts in the correction circuit can be calculated using equation (10) in chapter 0, but the correct setting is critical. If the correction circuit is trimmed for a longer cable than actually connected the circuit starts oscillating and the output current can reach undefined values which might damage brain tissue. It is indispensable to check the settings for the correct actual cable length before the electrode is positioned into the brain. In practice, a cable with the same capacity as the electrode cable is prepared with a 1MΩ resistor simulating the electrode. With that cable the cable capacity compensation is safely adjusted before each stimulation session.

The variation of R2 in equation (10) serves to trim to the cable length. In the circuit, this resistor corresponds to R7 and the potentiometer VR2.

It would be also possible to vary R6 in the circuit with a potentiometer but that would yield one serious disadvantage : The resistance of the slider of a potentiometer is unreliable. It can increase during the trim procedure or due to oxidation of the surfaces. This could change the adjustment of the correction circuit to one for a longer cable with the consequences discussed above. Only for this reason the actual position of the potentiometer was chosen. Here a bad slider contact would have opposite effects. The cable length would appear to be too short which would be save for the animal.

The correction circuit can be switched off by setting its amplification to zero using SW2.

12.3.3 Results

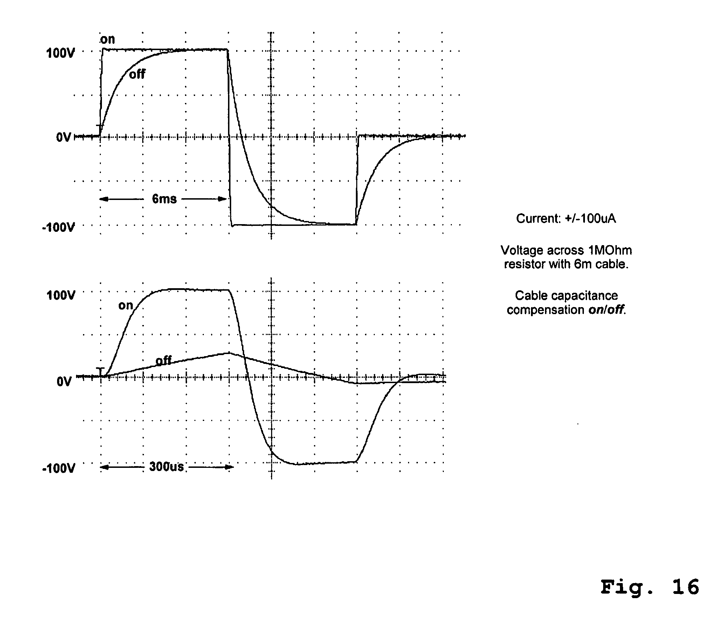

Figure 16 demonstrates the performance of the cable length compensation circuit. Biphasic pulses with +/-100μA were applied to a 1MΩ resistor simulating the electrode. The voltage rises to +/-100V. A cable of 6m length (the same length as in the stimulation experiments) was connected. With the correction circuit inactive the voltage and, consequently, the current through the 1MΩ resistor rises slowly according to the time constant formed by the resistor and the cable capacity. The rise time of about 2ms (upper diagram in figure 16) is much to long to generate pulses with the desired duration of 300μs (see lower diagram). With active and properly adjusted compensation circuit the rise time shrinks to about 100μs which is sufficient to generate the desired pulse duration (see lower diagram).

## Claims

1. Method of eliminating interfering currents in a measuring system being adapted for simultaneous recording electrophysiological signals and measuring fNMR images, said method comprising the steps of:

   - measuring electrophysiological current signals with an electrode device being in contact with a human or animal being,
   - converting the electrophysiological current signals into voltage signals with a current voltage converter, wherein the current voltage converter is subjected to an active interference compensation, and
   - recording an output of the current voltage converter as the electrophysiological signals to be investigated, said active interference compensation comprising the steps of:

     - measuring local magnetic field changes with a magnetic-field sensor for obtaining an interference reference signal being delivered to an compensation current injecting circuit, said local magnetic field changes being caused by alternating field gradients during fNMR image acquisition, and
- connecting an output of said compensation current injecting circuit with an input of the current voltage converter.

2. Method according to claim 1, wherein said local magnetic field changes are measured with at least one pick-up coil of said magnetic-field sensor.

3. Method according to claim 2, wherein said local magnetic field changes are measured with three identical, orthogonally-oriented coils of said detector electrode.

4. Method according to one of the foregoing claims, wherein said local magnetic field changes are measured in the vicinity of the electrode device.

5. Method according to one of the foregoing claims, wherein said local magnetic field changes are measured on gradient cables for supplying gradient coils being arranged for generating the alternating field gradients.

6. Measuring system being adapted for simultaneous recording electrophysiological signals and measuring fNMR images, said measuring system comprising:

- an electrode device for detecting electrophysiological current signals,
- a current voltage converter for converting the electrophysiological current signals into voltage signals,
- a magnetic-field sensor for measuring magnetic field changes and for obtaining an interference reference signal, said magnetic-field sensor being connected with an compensation current injecting circuit, wherein
- an output of said compensation current injecting circuit being connected with an input of the current voltage converter.

7. Measuring system according to claim 6, wherein said current voltage converter comprises an amplifier circuit as a first stage and a PI controller as a second stage.

8. Measuring system according to claim 6 or 7, wherein said magnetic-field sensor comprises at least one pick-up coil four measuring said local magnetic field changes.

9. Measuring system according to claim 8, wherein said magnetic-field sensor comprises three identical, orthogonally-oriented coils for measuring said local magnetic field changes.

10. Measuring system according to one of the claims 6 to 9, wherein said magnetic-field sensor is arranged in the vicinity of the electrode device.

11. Measuring system according to one of the claims 6 to 10, wherein said magnetic-field sensor is arranged on a gradient cable for supplying gradient coils.

**Patentansprüche**

1. Verfahren zur Beseitigung von Störströmen in einem Messsystems, das dazu angepasst ist, gleichzeitig elektrophysiologische Signale aufzuzeichnen und fNMR-Bilder zu messen, wobei das Verfahren die Schritte umfasst:

- Messen elektrophysiologischer Stromsignale mit einer Elektrodeneinrichtung, die in Kontakt mit einem menschlichen oder tierischen Wesen ist,
- Umwandeln der elektrophysiologischen Stromsignale in Spannungssignale mit einem Strom-Spannungs-Wandler, wobei der Strom-Spannungs-Wandler einer aktiven Störkompensation unterzogen wird, und
- Aufzeichnen eines Ausgangssignals des Strom-Spannungs-Wandlers als die zu untersuchenden elektrophysiologischen Signale, wobei die aktive Störkompensation die Schritte umfasst:

- Messen lokaler Magnetfeldänderungen mit einem Magnetfeldsensor, um ein Störreferenzsignal zu erhalten, das an eine Kompensationsstrominjektionsschaltung geliefert wird, wobei die lokalen Magnetfeldänderungen durch wechselnde Feldgradienten während der fNMR-Bilderfassung verursacht werden, und
- Verbinden eines Ausgangs der Kompensationsstrominjektionsschaltung mit einem Eingang des Strom-Spannungs-Wandlers.

2. Verfahren gemäß Anspruch 1, bei dem die lokalen Magnetfeldänderungen mit mindestens einer Abtastspule des Magnetfeldsensors gemessen werden.

3. Verfahren gemäß Anspruch 2, bei dem die lokalen Magnetfeldänderungen mit drei identischen, orthogonal orientierten Spulen der Detektorelektrode gemessen werden.

4. Verfahren gemäß einem der vorhergehenden Ansprüche, bei dem die lokalen Magnetfeldänderungen in der Nähe der Elektrodeneinrichtung gemessen werden.

**5.** Verfahren gemäß einem der vorhergehenden Ansprüche, bei dem die lokalen Magnetfeldänderungen auf Gradientenkabeln zur Versorgung von Gradientenspulen gemessen werden, die zur Erzeugung der alternierenden Feldgradienten angeordnet sind.

**6.** Messsystem, das angepasst ist, um gleichzeitig elektrophysiologische Signale aufzuzeichnen und fNMR-Bilder zu messen, wobei das Messsystem umfasst:

- eine Elektrodeneinrichtung zur Detektion elektrophysiologischer Stromsignale,
- einen Strom-Spannungs-Wandler zur Umwandlung der elektrophysiologischen Stromsignale in Spannungssignale,
- einen Magnetfeldsensor zur Messung magnetischer Feldänderungen und zur Erlangung eines Störreferenzsignals, wobei der Magnetfeldsensor mit einer Kompensationsstrominjektionsschaltung verbunden ist, wobei
- ein Ausgang der Kompensationsstrominjektionsschaltung mit einem Eingang des Strom-Spannungs-Wandlers verbunden ist.

**7.** Messsystem gemäß Anspruch 6, wobei der Strom-Spannungs-Wandler als eine erste Stufe eine Verstärkerschaltung und als eine zweite Stufe einen PI-Regler umfasst.

**8.** Messsystem gemäß Anspruch 6 oder 7, bei dem der Magnetfeldsensor mindestens eine Abtastspule zur Messung der lokalen Magnetfeldänderungen umfasst.

**9.** Messsystem gemäß Anspruch 8, bei dem der Magnetfeldsensor drei identische, orthogonal orientierte Spulen zur Messung der lokalen Magnetfeldänderungen umfasst.

**10.** Messsystem gemäß einem der Ansprüche 6 bis 9, bei dem der Magnetfeldsensor in der Nähe der Elektrodeneinrichtung angeordnet ist.

**11.** Messsystem gemäß einem der Ansprüche 6 bis 10, bei dem der Magnetfeldsensor auf einem Gradientenkabel zur Versorgung von Gradientenspulen angeordnet ist.

**Revendications**

**1.** Procédé d'élimination de courants d'interférence dans un système de mesure étant adapté pour l'enregistrement de signaux électrophysiologiques et la mesure d'images fNMR simultanés, ledit procédé comprenant les étapes consistant à :

- mesurer les signaux de courants électrophysiologiques avec un dispositif d'électrode étant en contact avec un être humain ou animal,
- convertir les signaux de courants électrophysiologiques en signaux de tension avec un convertisseur de courant tension, dans lequel le convertisseur de courant tension est soumis à une compensation d'interférence active, et
- enregistrer un sortie du convertisseur courant tension comme les signaux électrophysiologiques à investiguer, ladite compensation d'interférence active comprenant les étapes consistant à :

- mesurer des changements de champ magnétique local avec un capteur de champ magnétique pour obtenir un signal de référence d'interférence étant délivré à un circuit d'injection de courant de compensation, lesdits changements de champ magnétique local étant provoqués par des gradients de champ alternatifs pendant l'acquisition d'image fNMR, et
- connecter une sortie dudit circuit d'injection de courant de compensation avec une entrée du convertisseur de courant tension.

**2.** Procédé selon la revendication 1, dans lequel lesdits changements de champ magnétique local sont mesurés avec au moins une bobine de récupération dudit capteur de champ magnétique.

**3.** Procédé selon la revendication 2, dans lequel lesdits changements de champ magnétique local sont mesurés avec trois bobines identiques orientées de manière orthogonale de ladite électrode de détecteur.

**4.** Procédé selon l'une des revendications précédentes, dans lequel lesdits changements de champ magnétique local sont mesurés à proximité du dispositif d'électrode.

**5.** Procédé selon l'une des revendications précédentes, dans lequel lesdits changements de champ magnétique local sont mesurés sur des câbles de gradients pour alimenter des bobines de gradient étant agencées pour générer les gradients de champ alternatifs.

**6.** Système de mesure étant adapté pour l'enregistrement de signaux électrophysiologiques et la mesure d'images fNMR simultanés, ledit système de mesure comprenant :

- un dispositif d'électrode pour détecter des signaux de courants électrophysiologiques,
- un convertisseur courant tension pour convertir les signaux de courants électrophysiologiques en signaux de tension,
- un capteur de champ magnétique pour mesurer des changements de champ magnétique et pour obtenir un signal de référence d'interférence, ledit capteur de champ magnétique étant connecté avec un circuit d'injection de courant de compensation, dans lequel
- une sortie dudit circuit d'injection de courant de compensation étant connectée avec une entrée du convertisseur de courant tension.

7. Système de mesure selon la revendication 6, dans lequel ledit convertisseur de courant tension comprend un circuit d'amplificateur comme un premier étage et un contrôleur PI comme un deuxième étage.

8. Système de mesure selon la revendication 6 ou 7, dans lequel ledit capteur de champ magnétique comprend au moins une bobine de récupération pour mesurer lesdits changements de champ magnétique local.

9. Système de mesure selon la revendication 8, dans lequel ledit capteur de champ magnétique comprend trois bobines identiques orientées de manière orthogonale pour mesurer lesdits changements de champ magnétique local.

10. Système de mesure selon l'une des revendications 6 à 9, dans lequel ledit capteur de champ magnétique est agencé à proximité du dispositif d'électrode.

11. Système de mesure selon l'une des revendications 6 à 10, dans lequel ledit capteur de champ magnétique est agencé sur un câble de gradient pour alimenter les bobines de gradient.

Fig. 0.1

Magnetic Field Sensor

Current Sensor

Fig. 0.2

Fig. 0.3

**Gradient Currents**

1000 —

-1000 —

**Recorded Interference**

1000 —

-1000 —

1000 —

**Denoised Signal**

-1000 —

| | | | |
| 0 | 50 | 100 | 150 |

**Arbitrary (ADC) Units**

**Time in milliseconds**

Fig. 0.4

**Connection of the Monkey. Fig.1**

To RF-Amplifier

Rf-Resonance Circuit

RF-Coil

Eye Irrigating Specula

Silver wire for grounding liquids

Pumps for Irrigated Lid Specular

Infusion Pump

ECG-Monitor System

ECG-Pre-amplifier

Electrode Holder

Camber

In-Mouth Silver Wire

ECG-Elektrodes

Gradient Tube

Pick-Up Coils

Gradient Cables to Power Amplifiers

X
Y
Z

Oszilloscops, A to D Converter, etc.

Near Interference Compensation Circuit

Electrode I to V Converter

Far Interference Compensation Circuit

Output Stages with Isolation Amplifiers

Annular RF-Capacitors

Cdc 4.7uF

+ Input
−

Output

R1 330k
R2 330k

2m

2m

2m

4m

Green

R

EP 1 273 922 B1

# Record-Stimulation Control. Fig. 2

**Isolated TTL Input**

1k R1 · U1 TIL111 · D1

Off
Extern
Continous

**External Selection:**
Record or
Stimcurrent

Stim Control
Switch

**External and Manuell Switshing**

## Delay Circuit

+15V · +15V

100k R2

- U2
1 1/4 LM339
+ · R4 3k
1.5M R3 · 100n C1

- U3
1 2/4 LM339
+ · R6 3k
1.5M R5 · 100n C2

-15V

- U4 +15V
1 3/4 LM339
+ · -15V

100k R7

- U6
2 1/4 LM339
+

- U7
2 2/4 LM339
+

R8 10k · R9 10k · R10 10k

- U5
1 4/4 LM339
+

## Supply Supervisory Circuit

330k R11 · 8V
+15V · 47k R12 · - U8 +15V
47k R13 · 2 3/4 LM339
+ · -15V
D2 BZX79 7V5

+15V
1.5k R14
-7.5V
I to V
Conv. · 10k R15 · - U9
BZX79 6V2 D3 · 2 4/4 LM339
+
-15V · 1.8k R16

Q1 · Q2 · Q3
+15V
1/8 ULN2803A · 1/8 ULN2803A · 1/8 ULN2803A · -15V

R17 180 · +15V
680 R18
SDS-NAIS-Matsushita S2-24V

MEDER Electronic
MS12-1A71-75L

RL2 · RL1

## Mode Indicator

Colored mark

- U10
3 1/4 LM339
+ · 6.8k R19 · Record

- U11
3 2/4 LM339
+ · 6.8k R20 · Stim

+ U12 +15V
3 3/4 LM339
- · -15V · 6.8k R21 · Stim short-circuited

+15V · + U13
3 4/4 LM339
- · 6.8k R22

Stimulation
current source
or
Impedanz-
measurement

T2 · T1

Elektrode

Current to
Voltage
Converter

All integrated circuits have 100nF power supply by passing ceramic capacitor at each supply lead.
All resistors are 1% metalfilm TCO<=100ppm/K.

EP 1 273 922 B1

**Electrode Current to Voltage Converter. Fig. 3**

Stimulation-current or Impedanz-measurement

**Record Stimulation Switching**

To Record-Stimulation Control Circuit

RL1
RL2
T2
T1
Electrode
C1 15p
Near Interference Compensation Circuit

**Input Protection**

R4 20k
+15V
D3
D4
D1 BAS45
BAS45 D2
D6
D5
R5 20k

**Current to Voltage Converter** 10^7 V/A

R6 10M
C2 10n
R8 30k
+15V
OP37
-15V
U2
R7 2k
U1 AD743JN
+15V
-7.5V
R3 2k
R2 100
R1 1
Near Interference Compensation Circuit
To Record Stimulation Control Circuit

**Noise reduction circuit**

C3 10uF Tantal
R12 100k
R14 1M
R13 100k
-15V
R15 10k
U3 AD711JN
+15V
-15V
D7
R16 100
C4 100p
R17 10k
To Record Stimulation Control Circuit

**Gain x100**

+15V
Gm
U4 AD830AN
x1
Main Electrode Amplifier
-15V
Gm
R9 100
R11 1k
C5 2.2n
R10 10k
S1 1/10 Gain

All integrated circuits have 100nF power supply by passing ceramic capacitor at each supply lead.
All resistors are 1% metal film TCO<=100ppm/K.

33

## Comparision of Current to Voltage Converters. Fig. 4

Used 2-Stage Current-to-Voltage Converter

Voltage Gain

Proportional-Integral Controller

Classic Current-to-Voltage Converter

EP 1 273 922 B1

## Main Electrode Amplifier and Filter. Fig. 5

**3kHz Low-pass Filter**

Current to
Voltage
Converter

Low-pass filter, 3kHz,
3rd order Bessel
filter

**50mHz High-pass Filter**

High-pass filter,
50mHz,
2rd order
Bessel filter

Filter on/off

**Switch-selectable gain stage**

x1
x2
x3
x6
x10
x20
x30
x60
x100

**Strobe Signal Input**

1/2 HI-303

TIL111 U4

Isolated Strobe
signal input
to mask-off gradient
switching noise

**Output Isolation Amplifier**

ISO122JP

Electrode-
signal
Output

All integrated circuits have 100nF power supply by passing ceramic capacitor at each supply lead.
All resistors are 1% metalfilm TCO<=100ppm/K.

EP 1 273 922 B1

**Power Supply. Fig. 6**

Metal Case

**Main Isolated Supply**

280mVmean
860mVpeak

7815

C3 470n   C3 470n   +15V

C1 4700u

1 R6

B80C3700

2x15V
each
?A

LM320-15

470n C4   470n C4   -15V

C2 4700u

1 R7   257mVmean   870mVpeak

**Output Stages Supply**

+15V_iso

7815

C3 470n iso   C3 470n iso

C1 220u

iso

B80C1000

LM320-15

470n C4 iso   470n C4 iso   -15V_iso

C2 220u

2x15V
each
0.5A

Main
on/off

230VAC
Mains

# ECG-Preamplifier. Fig. 7

**ECG-Preamplifier**

Metal Case

**ECG-Methode II**

Red:
Right Wrist

Green:
Left Thigh

Yellow:
Ground of
Electrode
Holder

R1 330k    Red

R2 330k    Green

Yellow

C1 150n
R3 3.3M

High-pass
Time constant: 500ms
Cut-off Frequency: 320mHz

C2 150n    R4 3.3M

+15V
BAS45 D1
R5 2k    R7 2k
D2 BAS45
-15V

U1
AD711JN    +15V
-15V

R9 1k    D3  D5
D4  D6
4x BAS45

+15V
BAS45 D7
R6 2k    R8 2k
D8 BAS45
-15V

U2
AD711JN    +15V
-15V

R10 1k    D9  D11
D10  D12
4x BAS45

**External Isolated Power Supply**

230VAC
Mains

Main on/off

2x15V AC

C3 470u    C4 10u Tantal    C5 470n    7815    C6 470n    +15V

10u Tantal
470u C7    C8    470n C9    LM320 -15    470n C10    -15V

Red
Green
Yellow
R11 1k    Blue
Black
Guard

Hewlett
Packard
ECG
Plug-In
Modul
M1001A

-15V    4.7k R12    D13    -15V O.K.

+15V    4.7k R13    D14    +15V O.K.

Controller
Hewlett
Packard
Patient
Model 84
M1178A

Monitor
Hewlett
Packard
"Anesthesia"
Model:
M1094B

All integrated circuits have 100nF power supply
bypassing ceramic capacitor at each supply lead.
All resistors are 1% metalfilm TCO<=100ppm/K.

EP 1 273 922 B1

# "Far" Interference Compensation Circuit. Fig. 8

**Sensor Monitor Output**

**I to V Converter**

Electrodecable

Sensorcable

>>preamp

100k R4

C3 10n | R6 30k

U1 +15V OP27GP -15V

R5 2k

OP37 +15V -15V U2

300 R1 | 100p C1

R2 100

100n ker C2

D1 D2

R3 2k

300 R11 +15V U4 ISO122JP -15V | +15V_iso -15V_iso iso | 300 300 4.7n iso

**Gain x100**

Gm | +15V | U3 AD830AN | Gm | -15V

Electrode Holder
Grounding Tube
Recording chamber with 0.9% Saline
Sensor
Animal Head
"In mouth" Silver Wire
Cdc 4.7μ
Cel | Cgnd | Cse | Cmo | Cint
Neuronal Signal | Body fluids | "Far" Interference Source

**Output Stage**

+15V

R22 220 | R18 2.2k

U and I-Limiter
on/off

Q1 BC212B 10mA

D13 1N4448

D9 D11

R19 22k

D10 D12

R15 10k | R16 100k

+15V U5 AD817AN -15V

R17 1k

4x1N4448

D3 D6 | D4 D7 | D5 D8

6x1N4448

Q2 BC182B

R20 2.2k

R23 220 | R21 2.2k

-15V

D14 1N4448

100k R7

100n C5 | 1k R8 | 1k R9

100k R10 | 220p C4

**Proportional-Integral-Controller**

| | | | |
|---|---|---|---|
| 500 | 330n | 3x10M | 33u |
| 1.6k | 100n | 10M | 100u |
| 5k | 33n | 3M | 330u |
| 16k | 10n | 1M | 1m |
| 50k | 3.3n | 300k | 3.3m |
| 160k | 1n | 100k | 10m |
| 500k | 330p | 30k | 33m |
| 1.6M | 100p | 10k | 100m |
| 5M | 33p | 3k | 330m |
| 16M | 10p | 1k | 1 |
| 50M | 3.3p | 300 | 3.3 |

1k R24

Amplification

**DC-Bypass**

-12V D15

+15V U6 1/2 AD712 -15V

1μ C6

47k R14 | 10k R13 | 2.2n C7

| | |
|---|---|
| 10M | 16m |
| 3.3M | 50m |
| 1M | 160m |
| 330k | 500m |
| 100k | 1.6 |
| 33k | 5 |
| 10k | 16 |
| 3.3k | 50 |
| 1k | 160 |

DC-Cutoff frequency in Hz

Cut-off frequency in Hz

+15V 1/2 AD712 -15V U4

D16 -12V

D17 BZX79 12V

-12V R12 3k -15V

Cel Electrode Capacity
Cse Sensor Capacity
Cgnd Ground Capacity
Cmo Mouth to Silverwire Capacity
Cint Interference Source to Animal Coupling Capacity
Cdc DC-Current Decoupling

All integrated circuits have 100nF power supply bypassing ceramic capacitor at each supply lead. All resistors are 1% metalfilm TCO<=100ppm/K.

EP 1 273 922 B1

38

## Figure 8.1

**Theory of the "Near" Interference Compensation Circuit. Fig. 9** One Transformer Version 2

EP 1 273 922 B1

**Theory of the "Near" Interference Compensation Circuit. Fig. 10**   Tree Transformer Version 3

Rotational Asymmetry
of Electrode Holder
and/or the vicinity of
the electrode

+  I to V
−  Converter

Record
Electrode
Signal

Gradient
Coil

Neuronal
Signal

Cable
Capacity

Electrode

Summation
Stage

Variable
Gain
Amplifier

Pick-Up Coil
placed on
Gradient
cable

Gradient
Cable

TX1x

U1x

500p
C1

600p
C2

U2

U4

U3

Σ

+
+
+

U5x

TX2x

U6x

X Gradient
Power
Amplfier

TX1y

U1y

U5y

TX2y

U6y

Y Gradient
Power
Amplfier

TX1z

U1z

U5z

TX2z

U6z

Z Gradient
Power
Amplfier

EP 1 273 922 B1

## "Near" Interference Compensation Circuit. Fig. 11

1) RF blocking filter
2) Potentiometers Ten-Turns-Precision

All integrated circuits have 100nF power supply
bypassing ceramic capacitor at each supply lead.
All resistors are 1% metalfilm TCO<=100ppm/K.

## Figure 12

Fig. 13

**Voltage to Current Converter**

U1

$U_{ge}$ R1 $I_{in}$

Virtual Ground

**Electrode Cable**

$U_{out}$ C2

$I_{ca}$

$I_{out}$ $I_{el}$

**Electrode**

R4 C3

$I_{cor}$ $I_{di}$

C1

R2 R3

U2

$U_{di}$

**Correction Circuit**

Uge  Voltage of Pulsgenerator
Iin  Input Current
Uout Output Voltage of U1
Iout Output Current
Ica  Current in Cable Capacity
Iel  Current in Electrode
Idi  Input Current of Differentiator
Udi  Output Voltage of Differentiator
Icor Correction Current

**Fig. 14**

Fig. 15

Current: +/-100uA

Voltage across 1MOhm
resistor with 6m cable.

Cable capacitance
compensation *on/off*.

Fig. 16